# EUROPEAN PATENT SPECIFICATION

(11) **EP 2 475 361 B1**
(45) Date of publication and mention of the grant of the patent: **06.11.2019**
(21) Application number: 10759717.1
(22) Date of filing: 09.09.2010
(51) Int. Cl.: A61K 31/165, A61P 29/00

(54) **N-SUBSTITUTED BENZENEPROPANAMIDES FOR USE IN THE TREATMENT OF PAIN AND INFLAMMATION**
N-SUBSTITUIERTE BENZOLPROPANAMIDE ZUR BEHANDLUNG VON SCHMERZEN UND ENTZÜNDUNGEN
BENZÈNEPROPANAMIDES N-SUBSTITUÉS DESTINÉS À ÊTRE UTILISÉS DANS LE TRAITEMENT DE LA DOULEUR OU D'UNE INFLAMMATION

(30) Priority: 09.09.2009 US 240839 P
(43) Date of publication of application: 18.07.2012
(73) Proprietor: Novaremed Ltd., 4917001 Petah Tiqwa (IL)
(72) Inventor: KAPLAN, Eliahu, 49776 Petah Tiqwa (IL)
(74) Representative: Haile, Alison Victoria
(86) International application number: PCT/IB2010/002247
(87) International publication number: WO 2011/030205

(56) References cited:
- EP-A1- 1 876 169
- WO-A2-2004/031129
- WO-A2-2005/092305
- WO-A2-2007/129226
- WO-A2-2009/109850
- US-A1- 2008 194 672
- US-B1- 6 423 689

## Description

The present invention relates to the treatment or prophylaxis of pain and provides the use of certain compounds in the manufacture of medicaments for the treatment or prophylaxis of pain in humans and non-human animals.

Pain is a multifaceted or multidimensional, experiential response to a variety of stimulus conditions. Pain is defined by the International Association for the Study of Pain (IASP) as "an unpleasant sensory and emotional experience associated with actual or potential tissue damage, or described in terms of such damage".

Pain in animals is frequently the result of nociception, *i.e.,* activity in the nervous system that results from the stimulation of nociceptors. Neuropathic pain differs from nociceptive pain in that it involves damage to the nerve resulting in the sensation of pain. In central pain, the pain is generated in the brain from some form of lesion. Occasionally pain may be psychogenic, *i.e.,* caused by mental illness.

Pain can be acute or chronic. Acute pain is usually caused by soft tissue damage, infection and/or inflammation among other causes. Acute pain serves to alert after an injury or malfunction of the body. Chronic pain may have no apparent cause or may be caused by a developing illness or imbalance. Chronic pain is defined as the disease of pain; its origin, duration, intensity and specific symptoms may vary.

The experience of physiological pain can be grouped according to the source and related nociceptors. Cutaneous pain is caused by injury to the skin or superficial tissues. Cutaneous nociceptors terminate just below the skin, and due to the high concentration of nerve endings, produce a well-defined, localised pain of short duration. Examples of injuries that produce cutaneous pain include paper cuts, minor cuts, minor (first-degree) burns and lacerations. Somatic pain originates from ligaments, tendons, bones, blood vessels and nerves. It is detected with somatic nociceptors. The scarcity of pain receptors in these areas produces a dull, poorly-localised pain of longer duration than cutaneous pain; examples include sprains and broken bones. Myofascial pain is usually caused by trigger points in muscles, tendons and fascia and may be local or referred. Visceral pain originates from the body's viscera or organs. Visceral nociceptors are located within body organs and internal cavities. The even greater scarcity of nociceptors in these areas produces pain that is usually more aching and for longer duration than somatic pain. Visceral pain is extremely difficult to localise, and several injuries to visceral tissue exhibit "referred" pain, where the sensation is localised to an area completely unrelated to the site of injury. Phantom limb pain, a type of referred pain, is the sensation of pain from a limb that has been lost or for which a person no longer receives physical signals. Neuropathic pain may occur as a result of injury or disease to the nerve tissue itself. This can disrupt the ability of the sensory nerves to transmit correct information to the thalamus, and hence the brain interprets painful stimuli even though there is no obvious unknown psychological cause for the pain.

Acute pain is usually treated simultaneously with pharmaceuticals or appropriate techniques for removing the cause and pharmaceuticals or appropriate techniques for controlling the pain sensation, commonly analgesics.

Analgesics fall into three categories: opioid (narcotic) analgesics, non-opioid analgesics and adjuvant analgesics. Opioid analgesics are powerful analgesics that are chemically related to morphine. However, opioids have many side effects, which may be more likely to occur in people with certain disorders: kidney failure, a liver disorder, chronic obstructive pulmonary disease (COPD), dementia or another brain disorder. Drowsiness, constipation, nausea, vomiting and itching are common when opioids are started. Apart from morphine, opioid analgesics known at the time of writing include codeine, fentanyl, hydrocodone, hydromorphone, levorphanol, meperidine, methadone, oxycodone, oxymorphone, pentazocine and propoxyphene.

A variety of non-opioid analgesics are also available at the time of writing. They are often effective for mild to moderate pain. Most non-opioid analgesics are classified as non-steroidal anti-inflammatory drugs (NSAIDs). An example of an analgesic that is not an NSAID is acetaminophen, which is commonly known as paracetamol. Acetaminophen has substantially no anti-inflammatory properties.

NSAIDs are used to treat mild to moderate pain and may be combined with opioids to treat moderate to severe pain. NSAIDs not only relieve pain, but they also reduce the inflammation that often accompanies and worsens pain. Although widely used, NSAIDs can also have side effects, sometimes serious ones, including problems in the digestive tract, bleeding problems, problems related to retaining fluids and increased risk of heart and blood vessel disorders. Current NSAIDs include aspirin, ibuprofen, ketoprofen, naproxen, cox-2 inhibitors such as celecoxib, choline magnesium trisalicylate, diflunisal, salsalate, diclofenac, etodolac, fenoprofen, flurbiprofen, indomethacin, ketorolac, meclofenamate, mefenamic acid, meloxicam, nabumetone, oxaprozin, piroxicam, sulindac and tolmetin.

Adjuvant analgesics include antidepressants such, for example, as imipramine, amitriptyline, bupropion, desipramine, fluoxetine and venlafaxine; anticonvulsants (such as carbamazepine, gabapentin and pregabalin) and oral and topical local anaesthetics.

In the treatment of chronic pain, the "Three-Step Analgesic Ladder" developed by the World Health Organization is often used. For mild pain, acetaminophen, aspirin or other NSAIDs may be employed. For mild to moderate pain, week opioids such as codeine and dihydrocodeine are employed in combination with acetaminophen, aspirin or other NSAIDs. In the case of moderate to severe pain, strong opioids such as morphine, diamorphine, or fentanyl, hydromorphone, methadone, oxycodone or phenazocine may be administered in combination with acetaminophen, aspirin or other NSAIDs.

An object to the present invention is to provide alternative compounds for the treatment or prophylaxis of pain. In particular, it is object to the present invention to provide alternative NSAIDs for the treatment or prophylaxis of pain and to reduce inflammation. Desirably the compounds of the invention should have no or substantially no adverse activity on the central nervous system.
WO 2004/031129 relates to compounds useful in the treatment, prevention and control of immuno-allergical diseases, autoimmune diseases and organ or tissue transplantation rejection following transplantation. WO 2007/129226 relates to compounds for treating cell proliferative disorders. WO 2005/092305 relates to compounds for use in the treatment of viral diseases. Similar compounds have now been found useful in the treatment of pain.
According to one aspect of the present invention therefore there are provided compounds for use in the treatment or prevention of pain, which compounds are selected from the group consisting of: 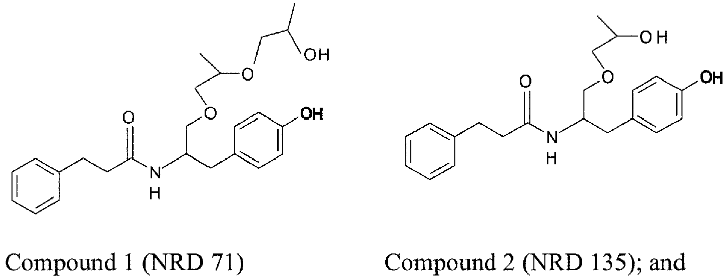 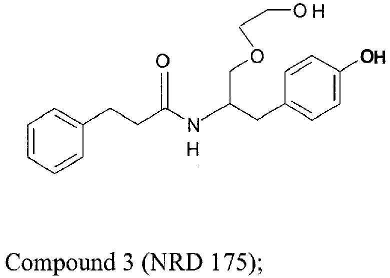 or a chiral, diastereomeric, racemic or geometric isomeric form thereof.

The compounds of the present invention may be used for the treatment or prophylaxis of acute or chronic pain. For instance, the compounds may be used for the treatment of nociceptive pain such, for example, as cutaneous pain, somatic pain, myofascial pain, visceral pain, phantom limb pain or neuropathic pain. The compounds of the invention may also be used in the treatment of headaches or migraine. The compounds may be used alone or in combination with acetaminophen or another NSAID for the treatment of mild chronic pain or in conjunction with weak or strong opioids for the treatment of moderate or severe pain.

The compounds of the invention may also be employed in the treatment or prophylaxis of neuropathic pain and may be used in conjunction with one or more antidepressants or antiepileptic medicaments such, for example, as gabapentin or pregabalin.

According to another aspect of the present invention there is provided a pharmaceutical composition for use in treating or preventing pain in a human or non-human animal patient, said composition comprising a pharmaceutically effective amount of one or more of the compounds of the invention.

Said one or more compounds may be in a pure, substantially pure or partially pure form as described in more detail below. The compounds may be administered under the supervision of a medical practitioner in an amount sufficient to achieve effective pain management. In some embodiments, the daily dose of said one or more compounds may be titrated to determine such effective amount.

Said one or more compounds may be administered parenterally, transdermally, intramuscularly, intravenously, intradermally, intranasally, subcutaneously, intraperitoneally, intraventricularly or rectally. Preferably, the one or more compounds are administered orally.

Optionally, the one or more compounds of the present invention may be administered simultaneously, sequentially or separately with at least one opioid analgesic, an antidepressant or an antiepileptic medicament. Alternatively, the one or more compounds of the invention may be administered simultaneously, sequentially or separately with one or more other NSAIDs or acetaminophen.

In yet another aspect of the present invention there is provided the use of one or more of the compounds of the invention in the manufacture of a medicament for use in the treatment or prophylaxis of pain. Said medicament may be manufactured for coadministration with one or more of acetaminophen, another NSAID, an opioid, an antiepileptic or an antidepressant.

Advantageously, it has been found that the compounds of the present invention are effective for reducing or preventing inflammation. It has also been found that the compounds of the invention have no or substantially no (*i.e.,* within acceptable limits) deleterious effect on the central nervous system.

All chiral, diastereomeric, racemic, and geometric isomeric forms of a structure are intended, unless specific stereochemistry or isomeric form is specifically indicated. For example, for compound 2, the following isomeric forms are intended:

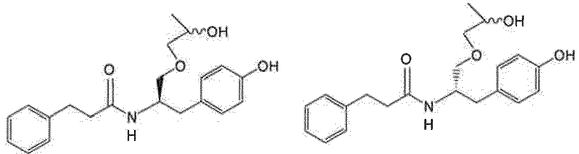

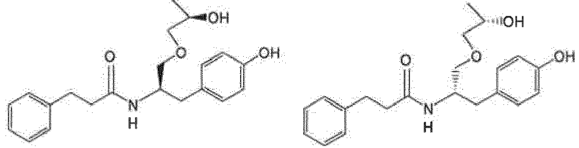

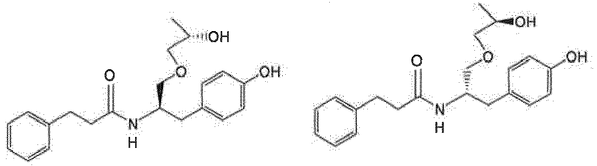

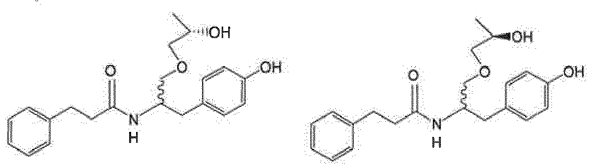

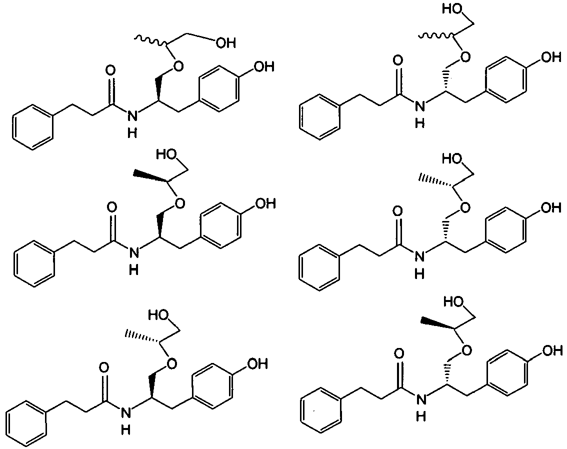

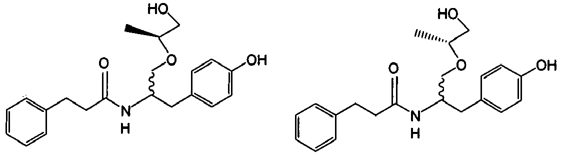

For example, some isomeric forms of compound 1 are shown below:

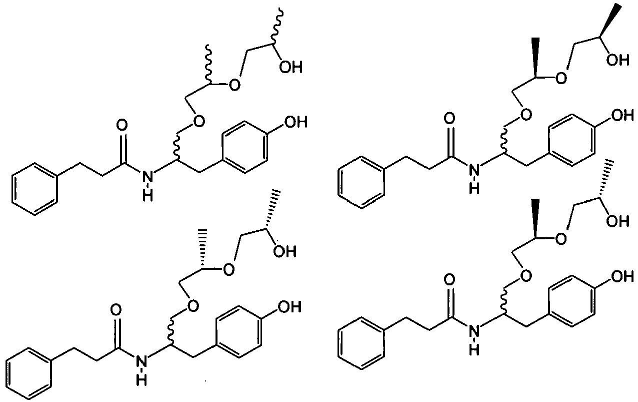

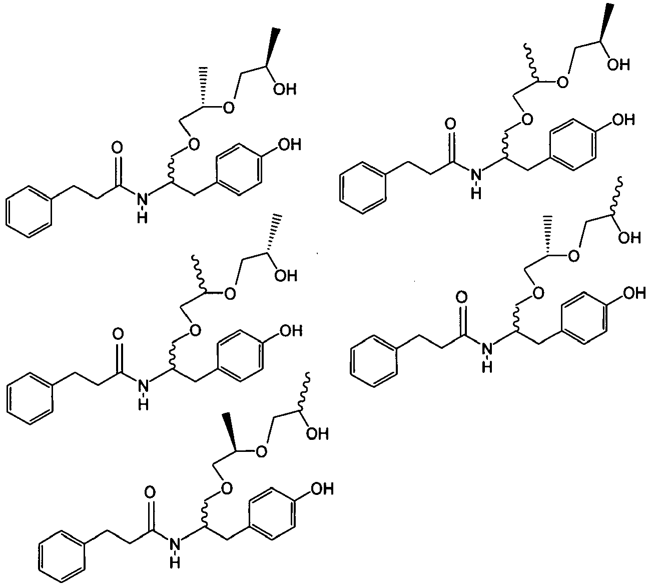

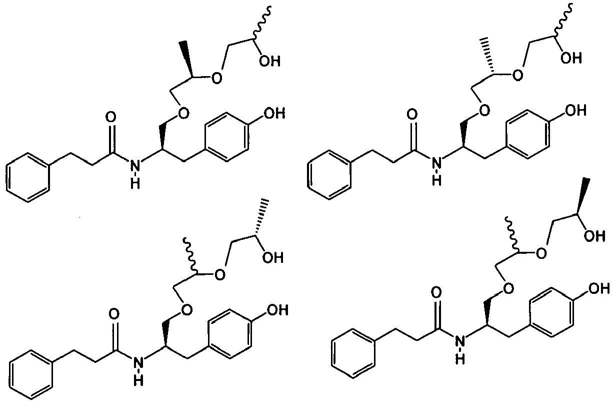

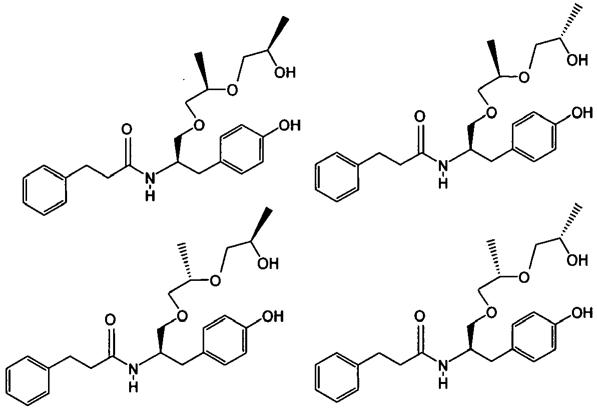

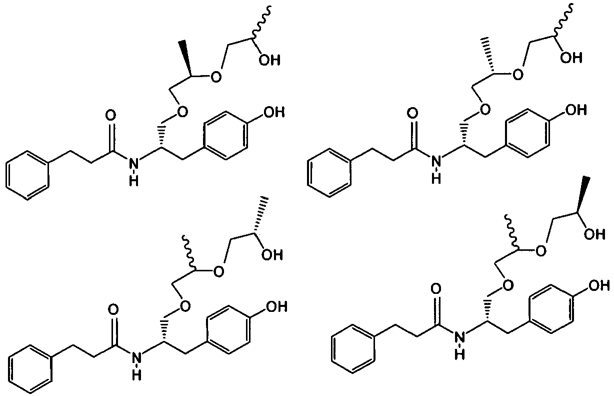

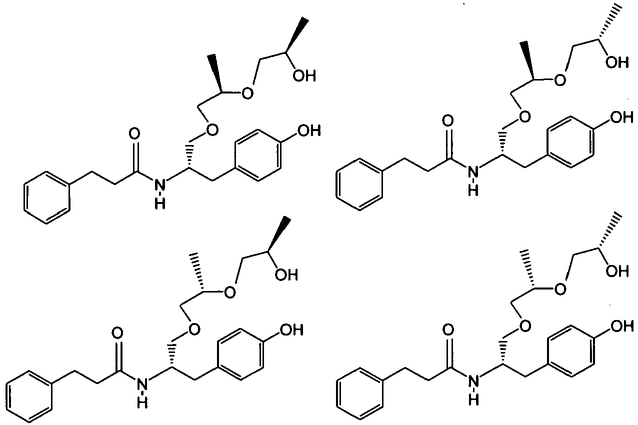

Isomeric forms of compound 1 also include geometric isomers as shown below, including all R and S permutations:

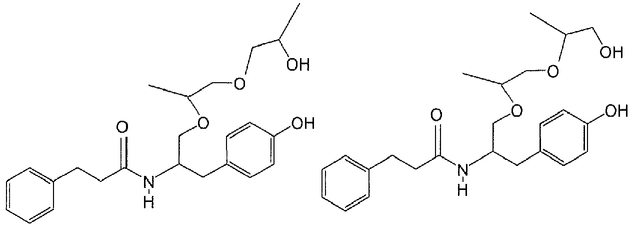

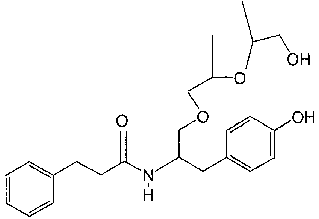

For example, some isomeric forms of compound 3 (NRD 175) are shown below:

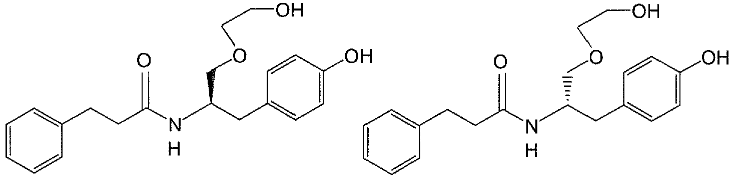

Suitable synthetic methods for obtaining and purifying the compounds of the present invention are disclosed in detail below. However, it should be apparent to a person skilled in the art that the compounds may be prepared using any other feasible synthetic methods.

The compounds of the invention may be synthesised as polyalkylene glycol (PAG) conjugates. Polymers that are used for such conjugation include poly(ethylene glycol) (PEG), also known as or poly(ethylene oxide) (PEO) and polypropylene glycol (including poly isopropylene glycol).

A polyalkylene glycol (PAG), such as PEG, is a linear polymer terminated at each end with hydroxyl groups:

HO-CH₂CH₂O-(CH₂CH₂O)ₚ-CH₂CH₂-OH.

The above polymer, α,ω-dihydroxyl poly(ethylene glycol), can also be represented as HO-PEG-OH, where it is understood that the -PEG- symbol represents the following structural unit:

-CH₂CH₂O-(CH₂CH₂O)ₚCH₂CH₂-

where p may range from 0 to about 48. PEG may be used as methoxy-PEG-OH, or mPEG, in which one terminus is the relatively inert methoxy group, while the other terminus is a hydroxyl group that is subject to ready chemical modification. Additionally, random or block copolymers of different alkylene oxides (*e.g*., ethylene oxide and propylene oxide) that are closely related to PEG in their chemistry may be substituted for PEG.

The PAG polymers may be linear or branched.

It is to be understood that compounds of the invention comprise a PAG moiety that may include a mixture of polymers which have a varying number of monomeric units. The synthesis of a PAG-conjugate compound may produce a population of molecules with a Poisson distribution of the number of monomeric units *per* polymer in the conjugate. Thus, a compound according to the invention that is described as having a polymer of n =2 monomeric units refers not only to the actual polymers in that population being described as having n = 2 monomeric units, but also to a population of molecules with the peak of the distribution being 2 or close to 2. The distribution of monomeric units in a given population can be determined, *e.g*., by nuclear magnetic resonance (NMR) or by mass spectrometry (MS).

In yet another aspect of the present invention there is provided a pharmaceutical composition for use in the treatment or prophylaxis of pain, said composition comprising a pharmaceutically effective amount of one or more of the compounds of the invention. Said composition may further comprise one or more pharmaceutically acceptable excipients. In some embodiments, said composition may also comprise acetaminophen, one or more other NSAIDs, one or more weak or strong opioids, an antidepressant or an antiepileptic agent.

The pharmaceutical composition of the invention may comprise one or more of the compounds of the invention in a pure, substantially pure or partially pure form. In some embodiments, said substantially pure form may comprise at least 95% wt. of said one or more compounds, *e.g.,* 96% wt., 97% wt., 98% wt. or more than 99% wt. of said compounds.

Said substantially or partially pure form of said compound(s) may further comprise a proportion of free polyalkylene glycol such, for example, as polyethylene glycol (PEG) or polypropylene glycol (PPG). Such polyalkylene glycol may itself be biologically active. The chain length of the free polyalkylene glycol may range from 1-50, preferably 1-25, more preferably 1 -5 or 1 or 2. In some embodiments, said polyalkylene glycol may have a chain length of 1, 2, 3 4 or 5 monomeric units. Said free polyalkylene glycol may comprise a mixture of different chain lengths. Thus, for a substantially pure form of said one or more compounds, said form may comprise up to 5% wt. of free polyalkylene glycol, e.g., up to 4% wt., 3% wt., 2% wt. or less than 1% wt., with the total amount in said form of said one or more compounds and said free polyalkylene glycol being 100% wt.

Said partially pure form of said one or more compounds may comprise about 5-60% wt. of the one or more compounds according to the invention and about 95-40% wt. of free polyalkylene glycol, the total amount being 100% wt.. Typically, said partially pure form may comprise about 45-55% wt. of said one or more compounds and about 55-45% wt. of said one or more polyalkylene glycols. Alternatively, said form may comprise about 80-95% wt. of said one or more compounds and about 20-5% wt. of said polyalkylene glycol(s).

Suitably, the composition of the invention may be formulated as a unit dosage form. Each unit dosage form may comprise all or a predetermined fraction of the daily dose amount of the one or more compounds of the invention, *e.g*., one half or one quarter of the daily dose amount.

Thus, the composition may be formulated as a tablet, a pill, a capsule, a powder, granules, a sterile parenteral solution or suspension, a metered aerosol or liquid spray, drops, an ampoule, an auto-injector device, a suppository, a cream or a gel. Said composition may be adapted for oral, enteral parenteral, intrathecal, intranasal, sublingual, rectal or topical administration, or for administration by inhalation or insufflation. Oral compositions such as tablets, pills, capsules or wafers are particularly preferred.

For preparing a solid dosage form such as a tablet, said one or more compounds may be mixed with one or more pharmaceutical excipients, *e. g*., conventional tabletting ingredients such as corn starch, lactose, sucrose, sorbitol, talc, stearic acid, magnesium stearate, dicalcium phosphate or gums, or other pharmaceutical diluents, *e. g.,* water, to form a solid pre-formulation composition containing a substantially homogeneous mixture of said one or more compounds, such that said one or more compounds are dispersed evenly throughout the composition, so that the composition may be readily subdivided into equally effective unit dosage forms such as tablets, pills and capsules.

Said solid pre-formulation composition is then subdivided into unit dosage forms of the kind mentioned above which may each contain from 0.1 to about 500 mg of the one or more compounds. Favoured unit dosage forms contain from 1 to 500 mg, *e.g.,* 1, 5, 10, 25, 50, 100, 300 or 500 mg, of the compound(s).

When formulated as a tablet or pill, said tablet or pill may be coated or otherwise compounded to provide a dosage form affording the advantage of prolonged action. For instance, said tablet or pill can comprise an inner dosage and an outer dosage component, the latter being in the form of an envelope over the former. These two components may be separated by an enteric layer that serves to resist disintegration in the stomach and permits the inner component to pass intact into the duodenum or to be delayed in release. A variety of materials are known in the use in such enteric layers or coatings, such materials including a number of polymeric acids and mixtures of polymeric acids with such materials as shellac, cetyl alcohol and cellulose acetate.

Alternatively, the pharmaceutical composition of the present invention may be formulated as a liquid dosage form for administration orally or by injection; for example an aqueous solution, a suitably flavoured syrup, an aqueous or oil suspension or a flavoured emulsion with edible oils such, for example, as cottonseed oil, sesame oil, coconut oil or peanut oil, as well as an elixir or a similar pharmaceutical vehicle. Suitable dispersing or suspending agents for an aqueous suspension include synthetic and natural gums, e.g., tragacanth, acacia, alginate, dextran, sodium carboxymethylcellulose, methylcellulose, polyvinylpyrrolidone or gelatin.

The following is a description by way of example only with reference to the accompanying drawings of embodiments of the present invention.

In the drawings:
FIG. 1a provides graphs showing the results (time of reaction vs. time after treatment) of a Hot-Plate Test using Balb/c mice following administration of Compound 2 or 3 of the invention;
FIG. 1b provides graphs showing the results (total time vs. formulation) of a Hot-Plate Test using Balb/c mice following administration of Compound 2 or 3 of the invention;
FIG. 1c provides graphs showing the results (weight of organs vs. formulation) of a Hot-Plate Test using Balb/c mice following administration of Compound 2 or 3 of the invention;
FIG. 1d provides a graph showing the results (time of reaction vs. formulation) of a Hot-Plate Test using Balb/c mice following administration of Compound 2 or 3 of the invention;
FIG. 2a provides graphs showing the results (time of reaction vs. time after treatment) of a Hot-Plate Test using Balb/c mice following administration of Compound 2 of the invention;
FIG. 2b provides graphs showing the results (total time vs. formulation) of a Hot-Plate Test using Balb/c mice following administration of Compound 2 of the invention;
FIGs. 3a and 3b provide graphs showing the results (time of reaction vs. time after treatment) of a Hot-Plate Test using Balb/c mice following administration of Compound 2 of the invention;
FIG. 3c provides a graph showing the results (total time vs. formulation) of a Hot-Plate Test using Balb/c mice following administration of Compound 2 of the invention;
FIG. 4a provides a graph showing the results (time of reaction vs. time after treatment) of a Hot-Plate Test using Balb/c mice following administration of Compound 2 of the invention;
FIG. 4b provides a graph showing the results (total time vs. formulation) of a Hot-Plate Test using Balb/c mice following administration of Compound 2 of the invention;
FIG. 5a provides a graph showing the results (time of reaction vs. time after treatment) of a Hot-Plate Test using Balb/c mice following administration of Compound 2 or 3 of the invention;
FIG. 5b provides a graph showing the results (total time vs. formulation) of a Hot-Plate Test using Balb/c mice following administration of Compound 2 or 3 of the invention;
FIGs. 6a, 6b, and 6c provide graphs showing the results (time of reaction vs. time after treatment) of a Hot-Plate Test using Balb/c mice following administration of Compound 2 of the invention;
FIGs. 6d and 6e provide graphs showing the results (total time vs. formulation) of a Hot-Plate Test using Balb/c mice following administration of Compound 2 of the invention;
FIGs. 7a, and 7b provide graphs showing the results (time of reaction vs. time after treatment) of a Hot-Plate Test using Balb/c mice following administration of Compound 1 of the invention;
FIGs. 7c and 7d provide graphs showing the results (total time vs. formulation) of a Hot-Plate Test using Balb/c mice following administration of Compound 1 of the invention;
FIG. 8a provides a graph showing the results (time of reaction vs. time after treatment) of a Hot-Plate Test using Balb/c mice following administration of Compound 3 of the invention;
FIG. 8b provides a graph showing the results (total time vs. formulation) of a Hot-Plate Test using Balb/c mice following administration of Compound 3 of the invention;
FIGs. 9a, and 9b provide graphs showing the results (time of reaction vs. time after treatment) of a Hot-Plate Test using Balb/c mice following administration of Compound 1 or 2 of the invention;
FIGs. 9c, 9d, 9e provide graphs showing the results (total time vs. formulation) of a Hot-Plate Test using Balb/c mice following administration of Compound 1 or 2 of the invention;
FIGs. 10a and 10b provide graphs showing the results (time of reaction vs. time after treatment) of a Hot-Plate Test using Balb/c mice following administration of Compound 1 or 2 of the invention;
FIG. 10c provides a graph showing the results (total time vs. formulation) of a Hot-Plate Test using Balb/c mice following administration of Compound 1 or 2 of the invention;
FIG. 11 provides a graph showing the results (increased volume vs. formulation) of an edema test of SD rats following administration of Compound 2 or 3 of the invention;
FIGs. 12a-12e provide graphs showing the results (reaction vs. formulation) of a formalin test on Balb/c mice following administration of Compound 1 or 2 of the invention.

### Synthesis of Polyalkylene Glycol Compounds

Polyalkylene glycol compounds were generally synthesised by preparation of the appropriate alcohol compound followed by conjugation of the alcohol with a polyalkylene glycol (PAG) polymer (*e.g*., polyethylene glycol (PEG) or polypropylene glycol (PPG)) of the desired length.

Compounds A to E and G to L are compounds of the disclosure. Compounds F, M and N are used in the production of compounds of the invention.

### Synthesis 1: Compound A (phenyl alaninol)

1.2 g, 32 mM, of LiAlH₄ were added to 2.3 g, 10 mM, phenyl alanine ethyl ester HCl in 50 ml dry ether. After stirring for 2 hours at room temperature, water and KOH were added and the reaction product was extracted with ethyl acetate. After evaporation, 0.8 g of Compound A, a light yellow oil, was obtained.

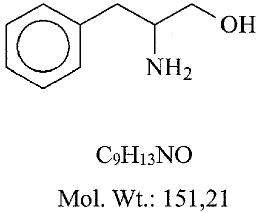

Compound **A** crystallised on standing. Mp-70.
NMR CDCl₃ 7.30(5H,m), 3.64(1H,dd,J=10.5,3.8 Hz) 3.40(1H,dd,J=10.5,7.2 Hz) 3.12(1H,m), 2.81(1H,dd,J=13.2, 5.2 Hz), 2.52(1H, dd, J=13.2, 8.6 Hz)
NMR acetone d₆ 7.30(5H, m), 3.76(1H, dt) 3.60(1H, m) 3.30 (1H, t),2.85(2H, m). *Helv. Chim. Acta,* 31, 1617(1948). Biels.-E3,Vol. 13,p 1757.

### Synthesis 2: Compound B (tyrosinol)

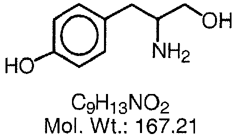

To 3 g, 12 mM, L- tyrosine ethyl ester HCl in 50 ml dry ether was added 1.2 g 32 mM LiAlH₄. After stirring 3 hours at room temperature, water and KOH were added and the reaction was extracted with ethyl acetate. Evaporation gave 1.1 g of a light yellow oil, 54% yield, which on standing crystallized. mp-85.
NMR CDCl₃ 7.20(4H,AB q, J=8.6 Hz), 3.50(2H,m) 3.20(1H,m), 2.81(2H,m).
NMR tyrosine ethyl ester free base CDCl₃ 7.0,6.56(4H, AB q, J=8.8 Hz), 4.20(2H, q, J=7, 0 Hz), 3.70, 3.0, 2.80(3H, 12 line ABXm), 1.28. (3H, t, J=7.0 Hz). JACS 71, 305(1949). Biels. -E3, Vol. 13, p 2263.

### Synthesis 3: Compound C

0.66 g 4-hydroxy hydrocinnamic acid and 4 ml thionyl chloride in 30 ml cyclohexane were refluxed for 2 hours. After evaporation, a white solid was obtained, to which 0.65 g oil of Compound A (4.3 mM) in 30 ml dichloromethane and 0.4 ml triethyl amine were added. After stirring for 2 hours at room temperature, water and KOH were added in order to neutralize the pH. The reaction product was extracted with dichloromethane. Evaporation gave 0.8 g of Compound C, light yellow viscous oil. Part of this product was triturated and recrystallized with ethanol to give a white solid. Mp-149.
NMR CDCl3 7.30-6.9(9H, m), 3.50(2H, m) 3.30(2H, t, J=7.2 Hz) 2.90 (3H, m), 2.60(2H, t, J=7.2 Hz).

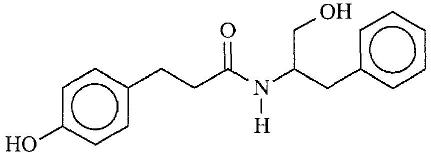

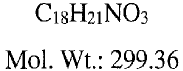

### Synthesis 4: Compound D

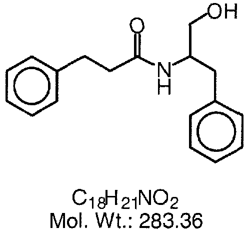

0.75 g, 5mM, hydrocinnamic acid and 4 ml thionyl chloride in 30 ml cyclohexane were refluxed for 2 hours. Evaporation gave a white solid to which were added 0.83 g, 5.5 mM, phenyl alaninol in 30 ml dichloromethane and 0.5 ml triethyl amine. After stirring 3 hours at room temperature, water and KOH were added to neutral pH and the reaction was extracted with dichloromethane. Evaporation gave 0.57 g of a yellow viscous oil, 40% yield.
NMR CDCl₃ 7.40-7.10(10H, m), 3.60(2H, m) 3.35(2H, t, J=7.2 Hz) 2.95 (3H, m), 2.50(2H, t, J=7.2 Hz).

### Synthesis 5: Compound E

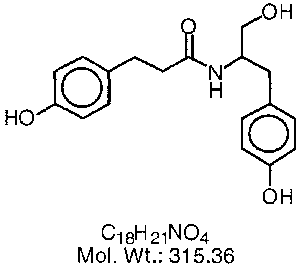

0.66 g, 4mM, 4-hydroxy hydrocinnamic acid and 4 ml thionyl chloride in 30 ml cyclohexane were refluxed 3 hours. Evaporation gave a light yellow solid to which were added 0.72 g, 4.3 mM, tyrosinol in 30 ml dichloromethane and 0.5 ml triethyl amine. After stirring 3 hours at room temperature. water and KOH were added to neutral pH and the reaction was extracted with dichloromethane. Evaporation gave 0.53 g light yellow viscous oil, 42% yield.
NMR CDCl₃ 7.30, 7.20 (8H, 2 ABq, J=8.6 Hz), 3.40(2H, m) 3.30(2H, t, J=7.2 Hz) 2.90 (3H, m), 2.60(2H, t, J=7.2 Hz).

### Synthesis 6: Compound F

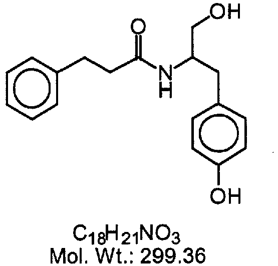

0.45 g, 3mM, hydrocinnamic acid and 3 ml thionyl chloride in 30 ml cyclohexane were refluxed for 2 hours. Evaporation gave a light yellow solid to which were added 0.58 g, 3.5 mM, tyrosinol in 30 ml dichloromethane and 0.4 ml triethyl amine. After stirring for 2.5 hours at room temperature, water and KOH were added to attain neutral pH and the reaction was extracted with dichloromethane. Evaporation gave 0.57 g light yellow viscous oil, 63% yield.
NMR CDCl₃ 7.40-7.10 (9H, m), 3.60(2H, m) 3.35 (2H, t, J=7.2 Hz) 2.95 (3H, m), 2.50 (2H, t, J=7.2 Hz).

### Compounds synthesised from phenyl alaninol (Compound A).

These compounds include those represented by the structure of formula **VIII:**

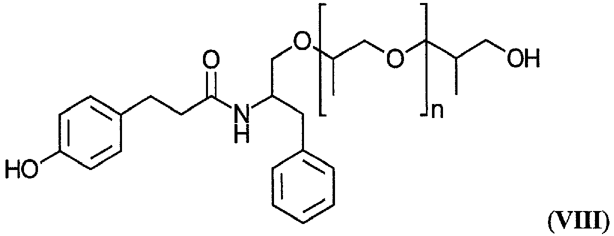

This compound can also be represented as formula A, where R is a polypropylene glycol polymer and n is the total number of polypropylene monomers in the polymer:

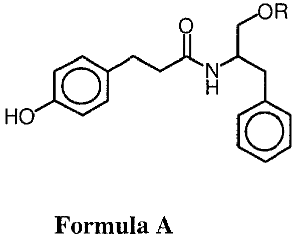

### Synthesis 7: Compound G

R=PPG (polypropylene glycol); n=2.

0.3 g Compound **C** (1mM), 0.8 g, 3 mM, triphenyl phosphine and 0.55 g 3.2 mM, ethyl diazo carboxylate were added to 1 g of poly (propylene glycol) (average mol. Weight 424 , n=7) in 60 ml dichloromethane. After stirring for 4 hours at room temperature, evaporation and chromatography gave 0.55 g viscous oil, a 73% yield. NMR CDCl₃ 7.30-6.9(9H, m), 4.1-3.0(m), 2.60(2H, t, J=7.2 Hz), 1.2-1.1(m). 0.1 g, 0.33 mmol of this product, potassium carbonate (0.069 g, 0.5 mmol, thinly crushed) and THF (3 mL, dried over KOH pellets) were put in a round-bottom flask equipped with a magnetic stirrer and a CaCl₂ drying tube. The mixture was cooled over an ice-salt bath (-10° C.) and a precooled solution of di-tert-butyldicarbonate (0.066 g, 0.30 mmole) in 2 mL THF (dried) was introduced dropwise. The mixture was allowed to stir at ice temperature for 1 hour and then for 2 days at room temperature. The reaction mixture was then evaporated, water (5 mL) introduced and the product was extracted with two 10 mL portions of ethyl acetate. The combined extracts were dried over anhydrous magnesium sulfate, paper-filtered and the solvent removed. The oily residue was triturated with a small amount of n-hexane and the solid formed recovered by vacuum filtration. Alternatively, the oily residue can be dissolved in an 1:1 mixture of ethyl acetate and hexane and the product recrystallised.

### Synthesis 8: Compound H

R=PPG; n=1.

Compound **H** was prepared using the same procedure as described above in Synthesis 7 for Compound **G,** with the substitution of the PPG, n=2 for PPG, n=1.

### Compounds synthesised from Compound D

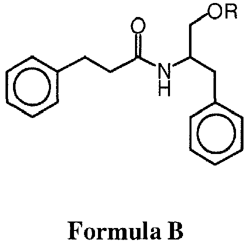

### Synthesis 9: Compound I

R=PPG; n=2.

Compound **I** was prepared as described above in Synthesis 7 from 0.22 g Compound **D.**

### Synthesis 10: Compound J

R=PPG; n=1.

Compound **J** was prepared as above in Synthesis 7 from 0.2 g Compound **D.**

### Compounds synthesised from Compound E (tyrosinol)

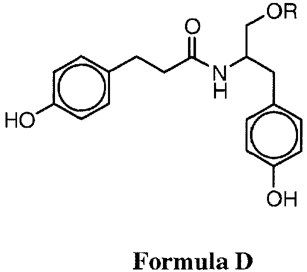

### Synthesis 11: Compound K

R=PPG; n=2.

Compound **K** was prepared as described above in Synthesis 7 from Compound **E.**

### Synthesis 12: Compound L

R=PPG; n=1

Compound **L** was prepared as described above in Synthesis 8 from Compound **E.**

### Compounds Synthesised from Compound F

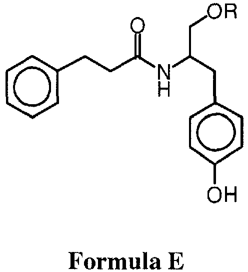

### Synthesis 13: Compound 1

R=PPG; n=2.

### Mesylation of PPG

106 mg of PPG₄₂₅ (0.25 mmol) was reacted with 90 mole-percent of mesyl chloride (26 mg, 2 drops) and 0.4 mmol pyridine (31.6 mg, 2 drops) to afford the mono-mesylated PPG (**M**). After combining PPG, mesyl chloride and pyridine, the mesylation reaction was carried out at 0° C. over 30 minutes while stirring, and then the reaction was continued for another 60 minutes at room temperature. During mixing, the reaction mixture turned from colorless to milky-white. The mixture was then dissolved in 5 mL methylene chloride and the organic phase was washed twice with 1M HCl solution, then twice with 1M NaOH

solution and once with water. The organic phase was dried over anhydrous sodium sulfate, filtered and the solvent removed.

### Sodium activation.

0.1g of compound F (0.25 mmol) was dissolved in 5 mL of absolute ethanol and then reacted with an equimolar amount of sodium ethoxide in absolute ethanol (previously prepared by reacting 0.25 mg-atom of sodium with an excess of absolute ethanol). The ethanol of the combined solutions was evaporated to total dryness to yield the sodium salt (**N**).

### Reacting M and N

**M** was dissolved in 5 mL of a potassium hydroxide-dried acetonitrile and the solution introduced into a round-bottom flask containing a magnetic stirrer. 5 mL of dried acetonitrile solution of **N** was introduced into the flask, followed by a catalytic amount (few crystals) of potassium iodide. A reflux condenser and a gas bubbler adjusted on top of it were connected to the reaction vessel and the reaction mixture was allowed to reflux under nitrogen atmosphere, while stirring, for 24 hrs. The reaction mixture was then paper-filtered and the solvent removed. The residue was dissolved in 2 mL of ethyl acetate and then passed through a silica-gel column, using ethyl acetate for elution. The TLC (elution with ethyl acetate) UV-absorbing spot at R_{f} = 0.55 turned out to contain the desired product **1** (a mixture of molecules containing different PPG sub-unit lengths), however, containing also some unreacted PPG. Other fractions contained unreacted mesylated PPG and doubly-mesylated PPG.

### Synthesis 14: Compound 2

R=PPG; n=1

Compound **2** was prepared using the same procedure as described above in Synthesis 13 from Compound F, with the substitution of the PPG, n=2 for PPG, n=1.

### Examples

The experiments described below were conducted to demonstrate the utility of compounds of the invention in the treatment of pain, using the following compounds.

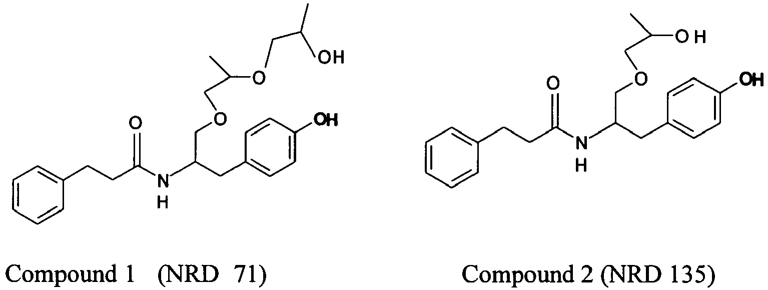

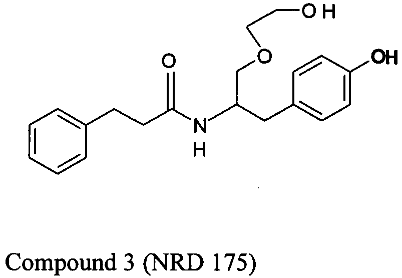

### Example 1

### Antinociceptive effect of Compound 2 and Compound 3

The objective of the study was to assess antinociceptive activity of tested items in the hot plate tests in mice, when administered sub-chronically. Measuring paw licking or jumping response time elapses following placement on heated surface (hot plate) was used to determine potential antinociceptive effect in mice.

A total of 42 Balb/c mice (12weeks old) were utilized. The mice were approximately 25g males at study initiation. The minimum and maximum weights of the group were within a range of ±10 % of group mean weight.

Compounds 2 and 3 were tested and compared with Diclofenac® (Perigo). DMSO solutions were used. Six groups of mice (each having *n=7 or n=8* mice) were tested, the last group receiving Diclofenac®.

| Animal group | Dose volume | Testing at: | Dose |
|---|---|---|---|
| | | Time/min | mg/kg/day |
| 1M (sham) (7) | 10ml/kg | -60, 0, 60, 120, 180, 240, 300, 360 | None |
| DMSO | | | |
| 2M+3M | | | 0.1; 5 |
| (7+7) | | | |
| compound 2 [1unit] | | | |
| 4M+5M (7+7) | | | 0.1; 5 |
| Compound 3 [1unit] | | | |
| 6M (8) | | | 10 |
| Diclofenac® | | | |

Formulations according to the following table were prepared for administration to the groups of mice.

| Formulation | Composition |
|---|---|
| 1 | Control - 0.02% DMSO 0.3ml/mouse, **po** (3 ml/10 mice) (0.6µl DMSO + 2999.4µl DDW), n=8. |
| 2 | Compound 2, 0.1 mg/Kg =0.003mg/0.3 ml/mouse, **po** (3ml/10 mice) (compound 2, 0.03 mg (Stock 50mg/ 1 ml DMSO) 0.6µl + 2999.4µl DDW), n=8 |
| 3 | Compound 2, 5 mg/Kg = 0.15 mg/0.3 ml/mouse, **po** (3ml/10 mice) (compound 2 1.5 mg( Stock 50 mg / 1 ml DMSO) 30 µl + 2970 µl DDW), n=8 |
| 4 | Compound 3, 0.1 mg/kg = 0.003mg/0.3 ml/mouse, **po** (3ml/10 mice) (compound 3, 0.03 mg (Stock 50mg/ 1 ml DMSO) 0.6µl + 2999.4µl DDW), n=8 |
| 5 | Compound 3, 5 mg/Kg = 0.15 mg/0.3 ml/mouse, **po** (3ml/10 mice) (compound 3 1.5 mg( Stock 50 mg / 1 ml DMSO) 30 µl + 2970µl DDW), n=8 |
| 6 | Diclofenac® 10 mg/kg = 0.3mg/0.3 ml/mouse, **po** (3ml/10 mice) from stock (Diclofenac® 51 mg + 51000 µl DDW), n=8 |

All groups received the drugs daily po for 16 days. Hot plate experiments were performed on days; 1, 8 and 15.

The following parameters were examined: body weight (days 1, 8 15); open field on day 16 including distance moved, velocity, immobility, rearings , time in center and other parameters. After the last experiment (i.e., open field day 16), animals were sacrificed by decapitation and blood was collected 24 hr after last drug administration. The following organs were dissected: liver (gall bladder), spleen, lungs, brain, heart and kidney for toxicity examination (formaldehyde 4%).

The hot plate is maintained thermostatically at a temperature of 52°C. One hour before the administration of the drugs, mice are tested in the hot plate. At time 0 the mice are administered with the test compound and the response to the hot plate is measured again at different times: 60,120,180, 240, 300 and 360 min.

Results are expressed as: Delta from maximum response [baseline vs. maximum response]; Absolute measures over time; and Accumulated time.

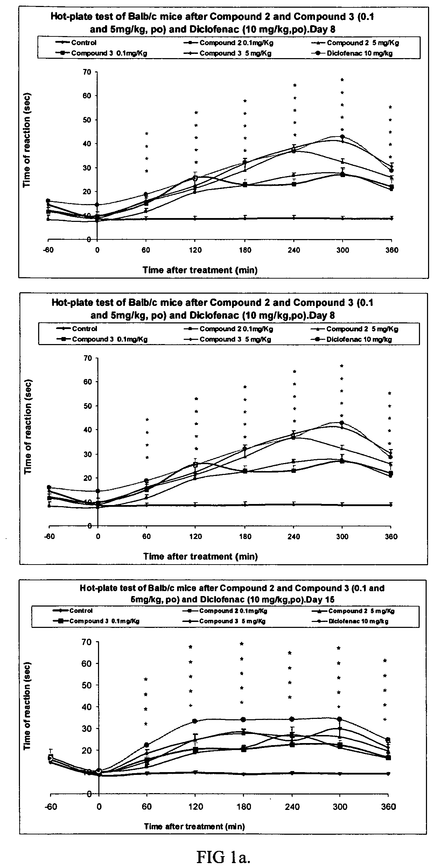

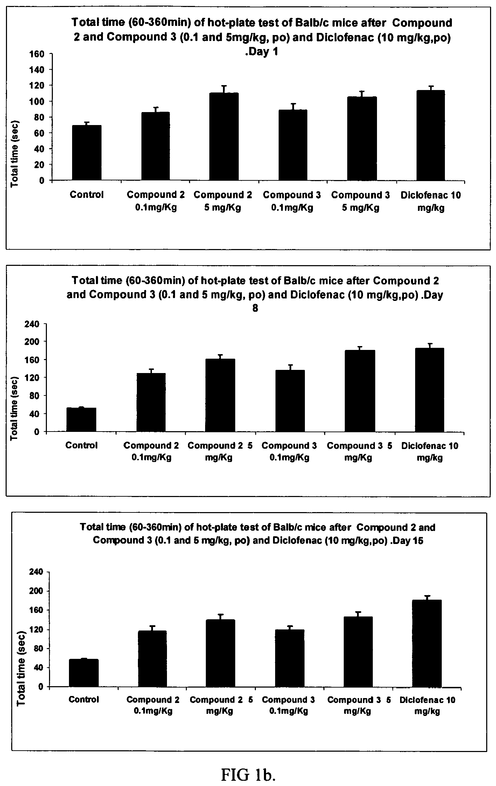

Figures 1a and 1b provide graphical results showing a comparison of compounds 2 and 3 with Diclofenac® at days 1, 8, and 15.

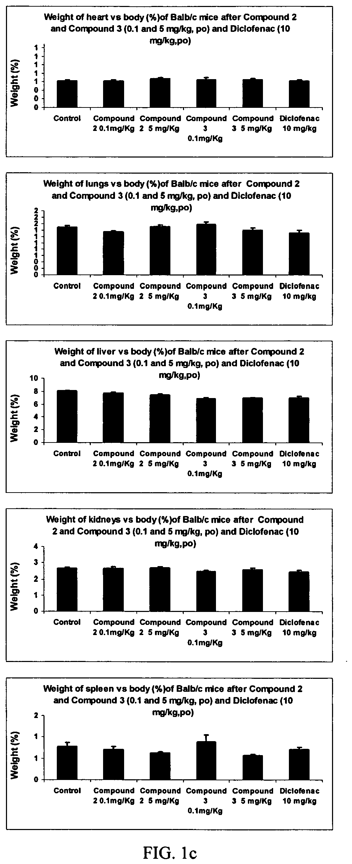

Figure 1c shows internal organ weight data after administration of the tests.

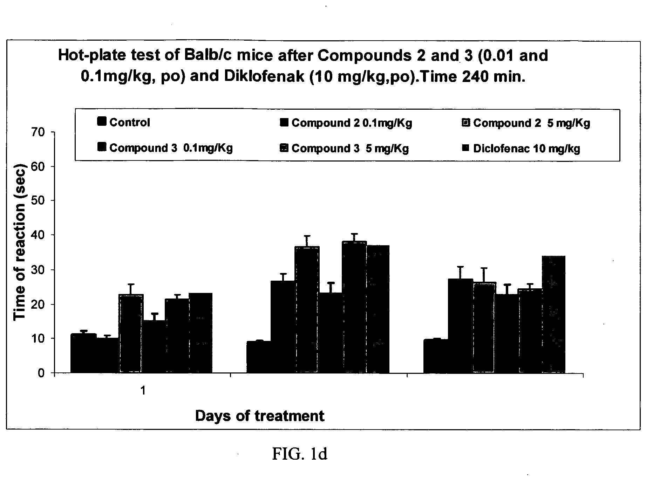

Additional data showed that compositions 2-5 significantly increased the time to reaction as compared with the control. A sample of such data is provided in FIG. 1d.

These data show that compounds 2 and 3 are effective as pain relievers.

### Example 2

### Nociceptin Activity using Compound 2

Using the procedure outlined in Example 1, 40 male mice (Balb/c, 9 weeks old, naive), were divided in 5 groups (8 mice per group) and treated daily (0 min, p.o.) with the formulations shown in the following table.

| Formulation | Composition |
|---|---|
| 1 | Control - 0.2% DMSO 0.3ml/mouse, po (3 ml/10 mice) (6 µl DMSO+ 2994 µl DDW), n=8. |
| 2 | Compound 2, 0.01 mg/Kg =0.0003mg/0.3 ml/mouse, po (3ml/10 mice) (Compound 2, 0.003 mg (Stock 50mg/ 1 ml DMSO) 0.06µl + 2999.94µl DDW), n=8 |
| 3 | Compound 2, 0.1 mg/Kg = 0.003 mg/0.3 ml/mouse, po (3ml/10 mice) (Compound 2, 0.03 mg(Stock 50 mg / 1 ml DMSO) 0.6 µl + 2999.4 µl DDW), n=8 |
| 4 | Compound 2, 1 mg/kg = 0.03mg/0.3 ml/mouse, po (3ml/10 mice) (Compound 2, 0.3 mg (Stock 50mg/ 1 ml DMSO) 6 µl + 2994 µl DDW), n=8 |
| 5 | Compound 2, 0.1 mg/Kg = 0.003 mg/0.3 ml/mouse, i.p. (3ml/10 mice) (Compound 2, 0.03 mg (Stock 50 mg / 1 ml DMSO) 0.6 µl + 2999.4 µl DDW), n=8 |

The animals were determined on the hotplate at: -60, 0, 120,240, 360,420 and 480 min. The hotplate mean temperature was 52 degrees ±1.

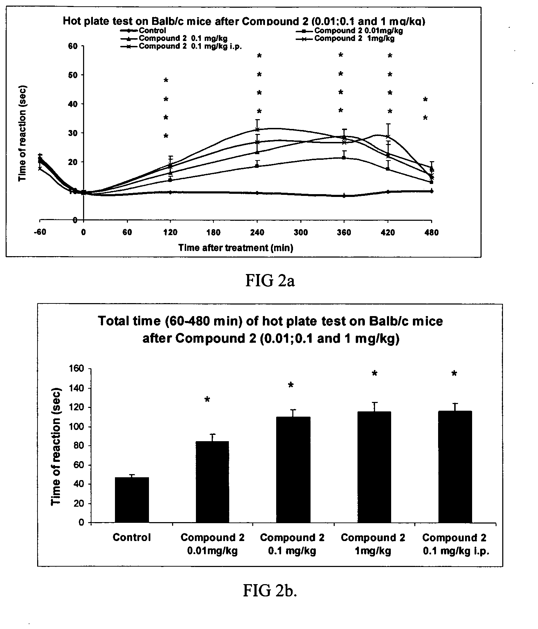

Figures 2a and 2b provide data for these tests.

### Example 3

### Nociceptin Activity using Compound 2

Using the procedure outlined in Example 1, 40 male mice (Balb/c, 9 weeks old, naive), were divided in 5 groups (8 mice per group) and treated daily (0 min, p.o.) with the formulations shown in the following table.

| Formulation | Composition |
|---|---|
| 1 | Control - DDW+2.5% DMSO 0.25 ml/mouse, po (2.5 ml/10 mice) (63 µl DMSO+ 2437 µl DDW ), n=8. |
| 2 | Compound 2, eqM 25 (12.5)mg/Kg =0.3125 mg/0.25 ml/mouse, po (2.5 ml/10 mice) ((Compound 2 3.125 mg (Stock 50mg/1ml DMSO) 63µl + 2437µlDDW) |
| 3 | Compound 2 eqM 12.5 (6.25)mg/Kg =0.15625mg/0.25 ml/mouse, po (2.5 ml/10 mice) ((Compound 2 1.5625 mg (Stock50mg/1ml DMSO) 32µl + 2468µl DDW) |
| 4 | Compound 2 eqM 6.25 (3.125)mg/Kg =0.078 mg/0.25 ml/mouse, po (2.5 ml/10 mice) ((Compound 2 0.78 mg (Stock50mg/1ml DMSO) 16µl + 2484µl DDW |

The animals were determined on HP at: -60, 0, 60,120, 180, 240, 300 and 360 min. The hot -plate means the temperature of 52 degrees ±1.

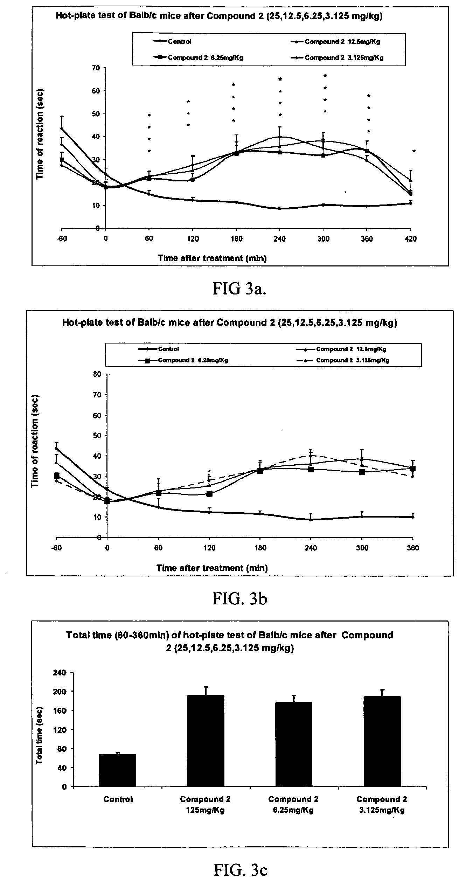

Figures 3a, 3b, and 3c provide data for this test.

### Example 4

### Nociceptin Activity using Compound 2

Using the procedure outlined in Example 1, 40 male mice (Balb/c, 13 weeks old, not naïve), were divided in 5 groups (8 mice per group) and treated daily (0 min, p.o.) with the formulations shown in the following table.

| Formulation | Composition |
|---|---|
| 1 | Control - DDW + 0.2% DMSO 0.3 ml/mouse, po (3 ml/10 mice) (6 µl DMSO + 2994 µl DDW), n=8. |
| 2 | Compound 2 (Mw 357) 1 mg/Kg =0.03mg/0.3 ml/mouse, po (3 ml/10 mice) ((Compound 2 0.3 mg (Stock50mg/1ml DMSO) 6 µl + 2994 µl DDW) |
| 3 | Compound 2 (Mw 357) 0.2 mg/Kg =0.006 mg/0.3 ml/mouse, po (3 ml/10 mice) ((Compound 2 0.06mg (Stock50mg/1ml DMSO) 1.2 µl + 2998.8 µl DDW) |
| 4 | Compound 2 (Mw 357) 0.04 mg/Kg =0.0012 mg/0.3 ml/mouse, po (3 ml/10 mice) ((Compound 2 0.012 mg (Stock 50mg/1ml DMSO) 0.24 µl + 2999.76 µlDDW) |
| 5 | Compound 2 (Mw 357) 0.008 mg/Kg =0.00024 mg/0.3 ml/mouse, po (3 ml/10 mice) ((Compound 2 0.0024 mg (Stock 50mg/1ml DMSO) 0.048(0.05) µl + 2999.95 µlDDW) |

The animals were determined on HP at: -60, 0, 60,120, 180, 240, 300 and 360 min after treatment. The hot-plate means the temperature of 52 degrees ±1.

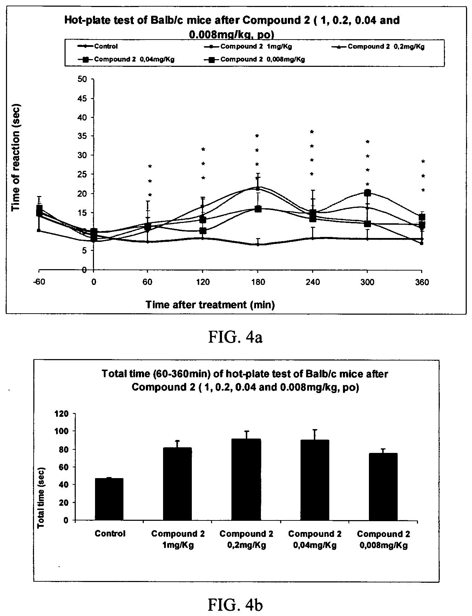

Figures 4a and 4b provide data for this test.

### Example 5

### Nociceptin Activity using Compound 2 and 3

Using the procedure outlined in Example 1, 40 male mice (Balb/c, 15 weeks old, not naïve), were divided in 5 groups (8 mice per group) and treated daily (0 min, p.o.) with the formulations shown in the following table.

| Formulation | Composition |
|---|---|
| 1 | Control - DDW + 0.02% DMSO 0.3 ml/mouse, po (3 ml/10 mice) (0.06 µl DMSO+ 2999.94 µl DDW), n=8. |
| 2 | Compound 2 (MW=357) 0.01 mg/Kg =0.0003 mg/0.3 ml/mouse, po (3 ml/10 mice) (Compound 2 0.003 mg (Stock 50 mg/1000 µl DMSO) 0.06 µl + 2999.94 µl DDW), n=8 |
| 3 | Compound 2 0.001 mg/Kg =0.00003 mg/0.3 ml/mouse, po (3 ml/10 mice) (Compound 2 0.0003 mg (Stock 50 mg/ 1000 µl DMSO) 0.006 µl + 2999.994 µl DDW), n=8 |
| 4 | Compound 3 (MW=343) 0.01 mg/Kg =0.0003 mg/0.3 ml/mouse, po (3 ml/10 mice)( Compound 3 0.003 mg (Stock 50 mg/ 1000 µl DMSO) 0.06 µl + 2999.94 µlDDW),n=8 |
| 5 | Compound 3 0.001 mg/Kg =0.00003 mg/0.3 ml/mouse, po (3 ml/10 mice)( Compound 3 0.0003 mg (Stock 50 mg/ 1000 µl DMSO) 0.006 µl + 2999.994 µlDDW),n=8 |

The animals were determined on HP at: -60, 0, 60,120, 180, 240, 300 and 360 min after treatment. The hot-plate means the temperature of 52 degrees ±1.

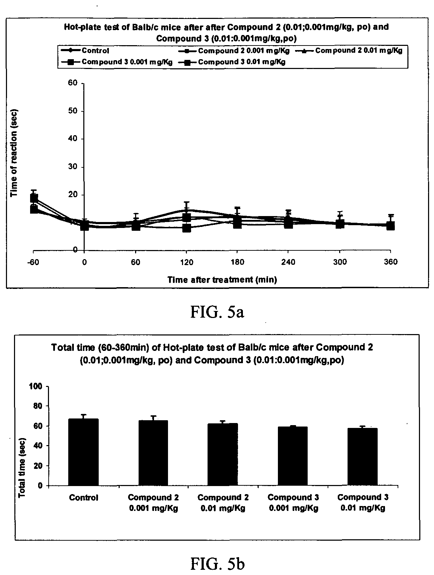

Figures 5a and 5b provide data for this experiment.

### Example 6

### Nociceptin Activity using Compound 2

Using the procedure outlined in Example 1, 40 male mice (Balb/c, 9 weeks old, not naive), were divided in 5 groups (8 mice per group) and treated daily (0 min, p.o.) with the formulations shown in the following table.

| Formulation | Composition |
|---|---|
| 1 | Control - DDW + 1.24% DMSO 0.25 ml/mouse, po (2.5 ml/10 mice) (31 µl DMSO+ 2469 µl DDW), n=8. |
| 2 | Compound 2 (Mw 357) 6.25 mg/Kg =0.15625mg/0.25 ml/mouse, po (2.5 ml/10 mice) ((Compound 2 1.5625 mg (Stock50mg/lml DMSO) 31.25 (31)µl + 2469µl DDW) |
| 3 | Compound 2 (Mw 357) 2.083 (2.1) mg/Kg =0.052mg/0.25 ml/mouse, po (2.5 ml/10 mice) ((Compound 2 0.52mg (Stock50mg/1ml DMSO) 10.415 (10)µl + 2490µl DDW) |
| 4 | Compound 2 (Mw 357) 0.694 (0.7) mg/Kg =0.01735 mg/0.25 ml/mouse, po (2.5 ml/10 mice) ((Compound 2 0.1735 mg (Stock 50mg/1ml DMSO) 3.47(3) µl + 2497µlDDW) |
| 5 | Gabapentine (GBP) 30mg/Kg = 7.5mg/0.25 ml/mouse, po (2.5 ml/10 mice) (GBP 7.5 mg+ 2500µlDDW) |

The animals were determined on HP at: -60, 0, 60,120, 180, 240, 300 and 360 min, and 24h after treatment. The hot-plate means the temperature of 52 degrees ± 1

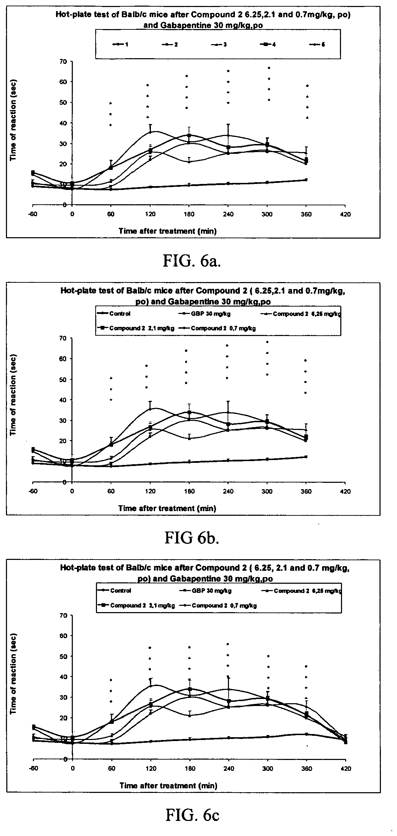

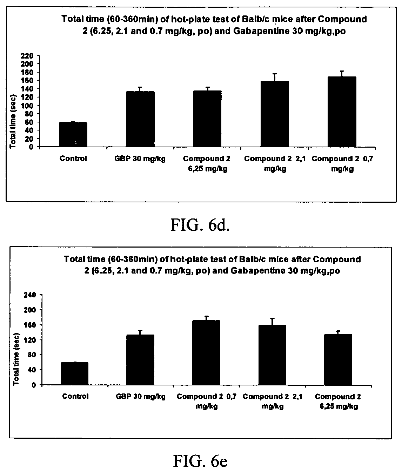

Figures 6a-e provide the data from this test.

### Example 7

### Nociceptin Activity using Compound 1

Using the procedure outlined in Example 1, 40 male mice (Balb/c, 12 weeks old, naive), were divided in 5 groups (8 mice per group) and treated daily (0 min, p.o.) with the formulations shown in the following table.

| Formulation | Composition |
|---|---|
| 1 | Control - DDW + 5% DMSO 0.25 ml/mouse, po (2.5 ml/10 mice) (125 µl DMSO + 2375 µl DDW), n=8. |
| 2 | Compound 1 25 mg/Kg = 0.625 mg/0.25 ml/mouse, po (2.5 ml/10 mice) (Compound 1 1.5625 mg (Stock 30mg/0.6 ml DMSO) 32µl + 2468µl DDW) |
| 3 | Compound 1 (Mw 415) eqM 25 mg/Kg (15 mg/kg) = 3.75 mg/0.25 ml/mouse, po (2.5 ml/10 mice)(Compound 1 3.75 mg (Stock 30mg/0.6ml DMSO) 75 µl + 2425µl DDW) |
| 4 | Compound 1 (Mw 415) eqM 12.5 mg/Kg (7.5 mg/kg) = 1.875 mg/0.25 ml/mouse, po (2.5 ml/10 mice)(Compound 1 1.875 mg (Stock 30mg/0.6 ml DMSO) 37.5 (38) µl + 2462µlDDW) |

The animals were determined on HP at: -60, 0, 60,120, 180, 240, 300 and 360 min, and 24h. The hot-plate means the temperature of 52 degrees ±1.

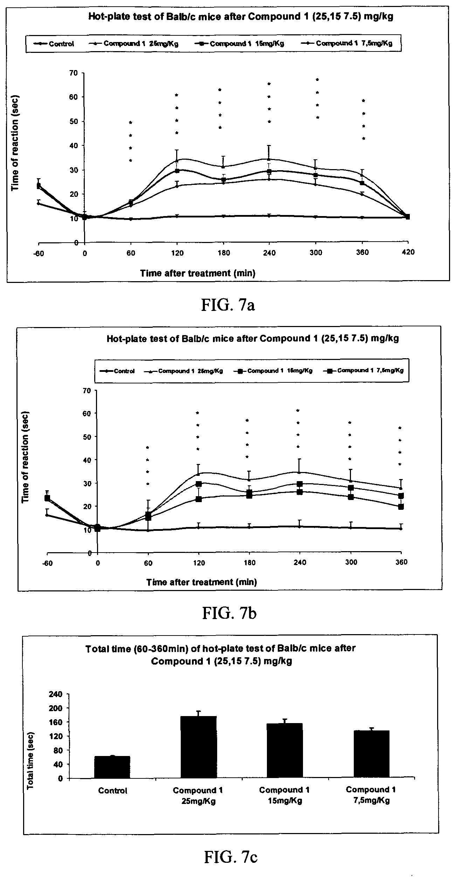

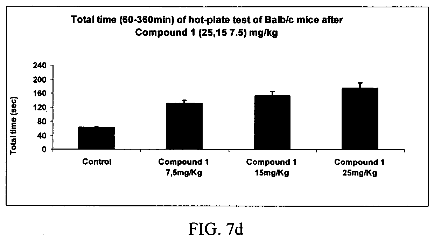

Figures 7a-d provide data for this experiment.

### Example 8

### Nociceptin Activity using Compound 3

Using the procedure outlined in Example 1, 37 male mice (Balb/c, 16 weeks old, not naïve), were divided in 5 groups and treated daily (0 min, p.o.) with the formulations shown in the following table.

| Formulation | Composition |
|---|---|
| 1 | Control - DDW + 1.2% DMSO 0.3 ml/mouse, po (3 ml/10 mice) (36 µl DMSO+ 2964 µl DDW), n=7. |
| 2 | Compound 3 (MW=343) 0.06 mg/Kg =0.0018 mg/0.3 ml/mouse, po (3 ml/10 mice) (Compound 3 0.018 mg (Stock 4.6 mg/92 µl DMSO) 0.36 µl + 2999.64 µl DDW), n=7 |
| 3 | Compound 3 0.6 mg/Kg =0.018 mg/0.3 ml/mouse, po (3 ml/10 mice) (Compound 3 0.18 mg (Stock 4.6 mg/ 92 µl DMSO) 3.6 µl + 2996.4 µl DDW), n=7 |
| 4 | Compound 3 6 mg/Kg =0.18 mg/0.3 ml/mouse, po (3 ml/10 mice) (Compound 3 1.8 mg (Stock 4.6 mg/ 92 µl DMSO) 36 µl + 2964 µlDDW), n=8 |
| 5 | Diclofenac 50 mg/Kg =1.25 mg/0.3 ml/mouse, po (3 ml/10 mice) Diclofenac 14.4 mg + 0.25 µl DMSO+ 2999.75 µlDDW), n=8 |

The animals were determined on HP at: -60, 0, 60,120, 180, 240, 300 and 360 min after treatment.The hot -plate means the temperature of 52 degrees ±1.

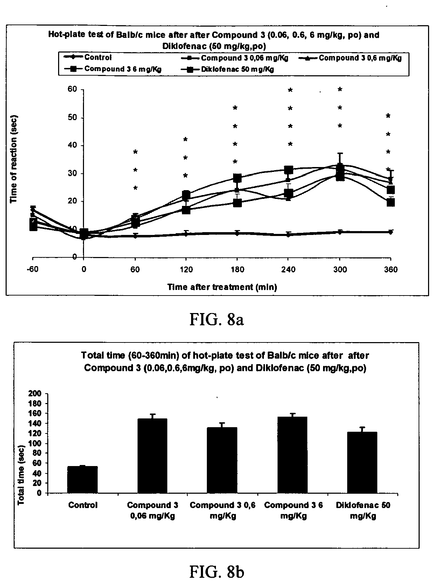

Figures 8a and 8b provide data for this experiment.

### Example 9

### Nociceptin Activity using Compounds 1 and 2

Using the procedure outlined in Example 1, 40 male mice (Balb/c, 13 weeks old, not naive), were divided in 5 groups and treated daily (0 min, p.o.) with the formulations shown in the following table.

| Formulation | Composition |
|---|---|
| 1 | Control - DDW + 0.8% DMSO 0.3 ml/mouse, po (3 ml/10 mice) (24 µl DMSO + 2976 µl DDW), n=8. |
| 2 | Compound 2 (Mw 357) 4 mg/Kg =0.12mg/0.3 ml/mouse, po (3 ml/10 mice) ((Compound 2 1.2 mg (Stock50mg/1ml DMSO) 24 µl + 2976 µl DDW) |
| 3 | Compound 2 (Mw 357) 0.04 mg/Kg =0.0012mg/0.3 ml/mouse, po (3 ml/10 mice) ((Compound 2 0.012 mg (Stock50mg/1ml DMSO) 0.24 µl + 2999.76 µl DDW) |
| 4 | Compound 1 (Mw 415) eq.4 (4.65)mg/Kg =0.14 mg/0.3 ml/mouse, po (3 ml/10 mice) ((Compound 1 1.395 mg (Stock 30mg/0.6ml DMSO) 27.9(28) µl + 2972 µlDDW) |
| 5 | Compound 1 (Mw 415) eq.0.04 (0.05)mg/Kg =0.0014 mg/0.3 ml/mouse, po (3 ml/10 mice) ((Compound 1 0.014 mg (Stock 60mg/0.6ml DMSO) 0.279 (0.28) µl + 2999.72µlDDW) |

The animals were determined on HP at : -60, 0, 60,120, 180, 240, 300 and 360 min after treatment. The hot -plate means the temperature of 52 degrees ±1.

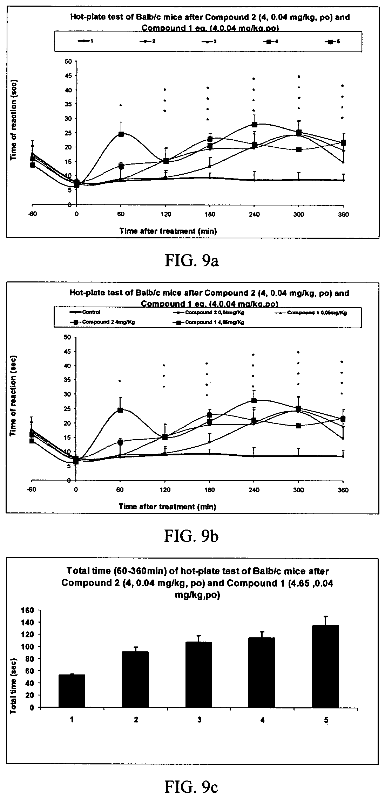

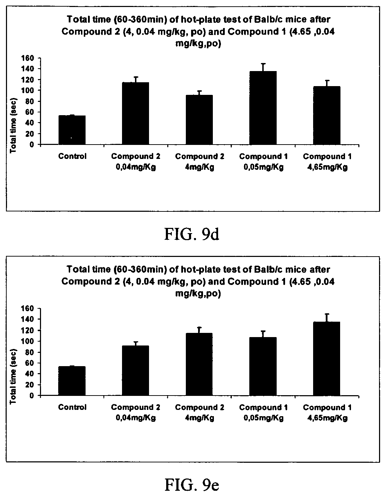

Figures 9a-e provide data for this experiment.

### Example 10

### Nociceptin Activity using Compounds 1 and 2

Using the procedure outlined in Example 1, 40 male mice (Balb/c, 13 weeks old, not naive), were divided in 5 groups and treated daily (0 min, p.o.) with the formulations shown in the following table.

| Formulation | Composition |
|---|---|
| 1 | Control - DDW + 0.04% DMSO 0.3 ml/mouse, po (3 ml/10 mice) (1.2 µl DMSO+ 2998.8 µl DDW), n=8. |
| 2 | Compound 1 0.125 mg/Kg =0.00375 mg/0.3 ml/mouse, po (3 ml/10 mice) (Compound 1 0.0375 mg (Stock 60mg/1.2 ml DMSO) 0.75µl + 2999.25 µl DDW), n=8 |
| 3 | Compound 2 0.1 mg/Kg =0.003 mg/0.3 ml/mouse, po (3 ml/10 mice) (Compound 2 0.03 mg (Stock 60mg/1.2ml DMSO) 0.6 µl + 2999.4 µlDDW), n=8 |
| 4 | Diclofenac 50 mg/Kg =1.25 mg/0.3 ml/mouse, po (3 ml/10 mice) Diclofenac 12.5 mg + 0.25 µl DMSO+ 2999.75µlDDW), n=8 |

The animals were determined on HP at : -60, 0, 60,120, 180, 240, 300 and 360 min after treatment. The hot -plate means the temperature of 52 degrees ±1.

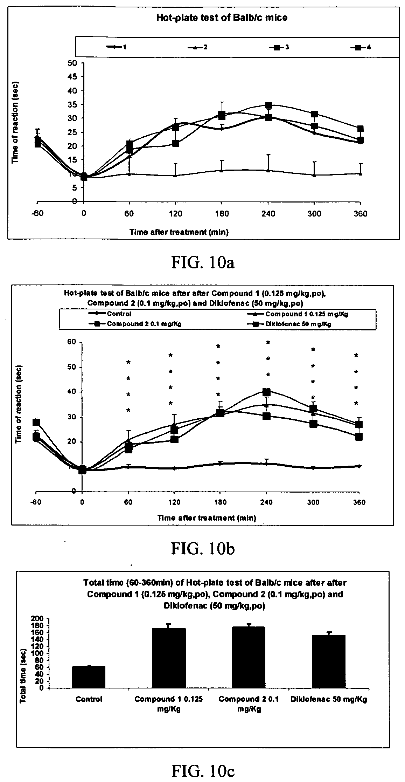

Figures 10a, 10b, and 10c provide data for this experiment.

### Example 11

### Edema test

Male SD rats (9 weeks old, naïve), were divided into 5 groups (6 mice in each group) and treated (-120 min, p.o.) with the formulations shown in the following table.

| Formulation | Composition |
|---|---|
| 1 | Control - DDW+10% DMSO 0.3 ml/rat, po (2.4 ml/8 rats) (241 µl DMSO+ 2159 µl DDW) |
| 2 | Compound 2, 1 mg/Kg = 0.3 mg/0.3 ml/rat, po (2.4 ml/8 rats) (Compound 2, 2.4 (Stock 21.5 mg/ 0.43 ml DMSO) 32 µl +2352 µl DDW) |
| 3 | Compound 2, 5 mg/Kg =1.5 mg/0.3 ml/rat, po (2.4 ml/8 rats) (Compound 2, 12 mg (Stock 21.5 mg/ 0.43 ml DMSO) 241 µl + 2159 µlDDW) |
| 4 | Compound 3 1 mg/Kg =0.3 mg/0.3 ml/rat, po (2.4 ml/8 rats) (Compound 3 2.4 mg (Stock 19.1 mg/ 0.382 ml DMSO) 32 µl + 2352 µl DDW) |
| 5 | Compound 3 5 mg/Kg =1.5 mg/0.3 ml/rat, po (2.4 ml/8 rats) (Compound 3, 12 mg (Stock 19.1 mg/ 0.382 ml DMSO) 241 µl + 2159 µl DDW) |

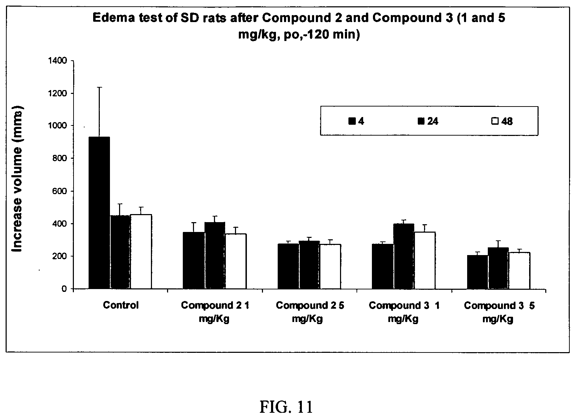

Figure 11 provides data from this test.

### Example 12

### Formalin Test

Formalin test. The method used was similar to that described by Hunscaar and Hole (1987) "The formalin test in mice: dissociation between inflammatory and non-inflammatory pain," Pain 30, pp.103-104.

Five animals are used in each group and two to three hours after oral administration of the conjugates, 40 µl or 20 µl (rats or mice, respectively) of a 1% formalin (in 0.9% saline) solution is injected subcutaneously into the dorsal surface hind paw. The formalin induced typical flinching behaviour of the injected paw was counted. The animals were returned to a glass chamber and the total time spent by the animal licking or biting the injected paw was measured. Formalin induced pain behaviour is biphasic. The duration of paw licking was determined during the following two time periods: 0-5 min (first-neurogenic phase) and 20-30 min (second-inflammatory phase) after formalin injection.

**Part a. Male mice (Balb/c mice, 27 weeks old, not naïve), were divided in 4 groups (5 mice per group) and treated (0 min, i.p.) with the following formulations, respectively:**

| Formulation | Composition |
|---|---|
| 1a | Control - (0.2 ml DMSO + 3.52 saline) i.p. 0.3 ml/ mouse. n=5. |
| 2a | Compound 2, 0.2 mg/kg = 0.006 mg/0.3 ml/mouse, 6 mice/ 0.036 mg/ 1.8 ml = (0.72 µl (2 mg compound 2 + 40 µl DMSO) + 1799.28 µl DDW) n=5. |
| 3a | Compound 2, 1 mg/kg = 0.03 mg/0.3 ml/mouse, 6 mice/0.18 mg/1.8 ml = (3.6 µl (2 mg compound 2 + 40 µl DMSO) + 1796.4 µl DDW) n=5. |
| 4a | Compound 2, 2.5 mg/kg = 0.15 mg/0.3 ml/mouse, 6 mice/0.9 mg /1.8 ml = (9 µl (2 mg stock compound 2 + 40 µl DMSO) + 1782 µl DDW) n=5. |

**Part b. Male mice (Balb/c mice, 27 weeks old, not naive), were divided in 4 groups (5 mice in groups) and treated (0 min, i.p.) with the following formulations, respectively:**

| Formulation | Composition |
|---|---|
| 1b | Control - (0.2 ml DMSO + 3.52 saline) i.p. 0.3 ml/ mouse. n=5. |
| 2b | Compound 2 0.2 mg/kg= 0.006 mg/0.3 ml/mouse, 6 mice/ 0.036 mg/ 1.8 ml = ( 0.72 µl (1.3 mg compound 2 + 26 µl DMSO) + 1799.28 µl DDW) n=5. |
| 3b | Compound 2 1 mg/kg = 0.03 mg/0.3 ml/mouse, 6 mice / 0.18 mg/ 1.8 ml = (3.6 µl (1.3 mg compound 2 + 26 µl DMSO) + 1796.4 µl DDW) n=5. |
| 4b | Compound 2 2.5 mg/kg = 0.15 mg/0.3 ml/mouse, 6 mice / 0.9 mg / 1.8 ml = (9 µl (1.3 mg compound 2 + 26 µl DMSO) + 1782 µl DDW) n=5. |

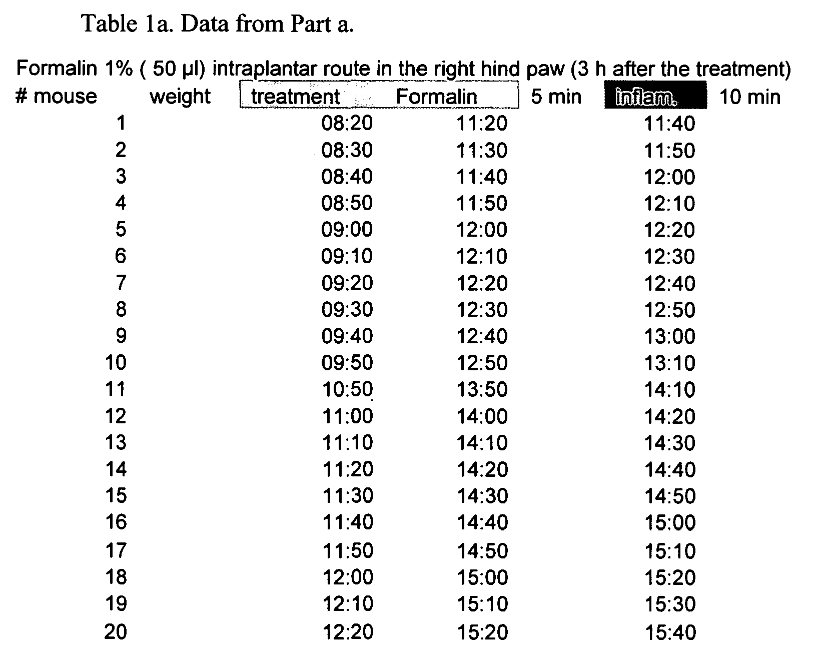

**Table 1b. Data from Part b.**

| Formalin 1% (50 µl) intraplantar route in the right hind paw (4 h after the treatment) | | | | | | |
|---|---|---|---|---|---|---|
| # mouse | weight | treatment | Formalin | 5 min | inflam. | 10 min |
| 1 | | 06:30 | 10:30 | | 10:50 | |
| 2 | | 06:40 | 10:40 | | 11:00 | |
| 3 | | 06:50 | 10:50 | | 11:10 | |
| 4 | | 07:00 | 11:00 | | 11:20 | |
| 5 | | 07:10 | 11:10 | | 11:30 | |
| 6 | | 07:20 | 11:20 | | 11:40 | |
| 7 | | 07:30 | 11:30 | | 11:50 | |
| 8 | | 07:40 | 11:40 | | 12:00 | |
| 9 | | 07:50 | 11:50 | | 12:10 | |
| 10 | | 08:00 | 12:00 | | 12:20 | |
| 11 | | 08:10 | 12:10 | | 12:30 | |
| 12 | | 08:20 | 12:20 | | 12:40 | |
| 13 | | 08:30 | 12:30 | | 12:50 | |
| 14 | | 08:40 | 12:40 | | 13:00 | |
| 15 | | 08:50 | 12:50 | | 13:10 | |
| 16 | | 09:00 | 13:00 | | 13:20 | |
| 17 | | 09:10 | 13:10 | | 13:30 | |
| 18 | | 09:20 | 13:20 | | 13:40 | |
| 19 | | 09:30 | 13:30 | | 13:50 | |
| 20 | | 09:40 | 13:40 | | 14:00 | |

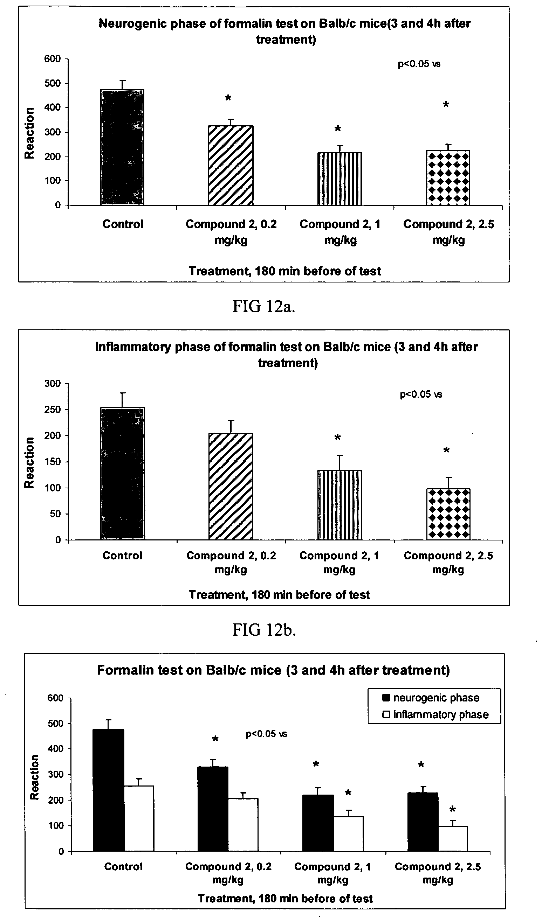

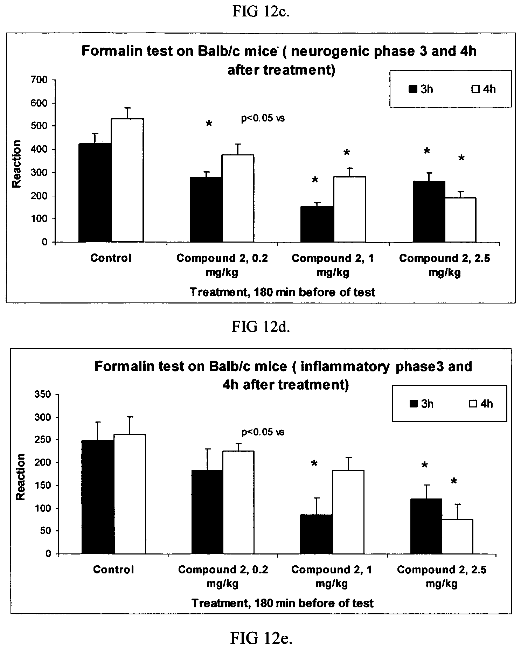

Results from these tests are plotted in Figures 12a and 12b respectively. Figures 12c, 12d, and 12e provide further data based on measurement time. These data further confirm the anti-inflammatory properties of Compound 2.

## Claims

1. A compound for use in the treatment or prophylaxis of pain, wherein the compound is selected from the group consisting of 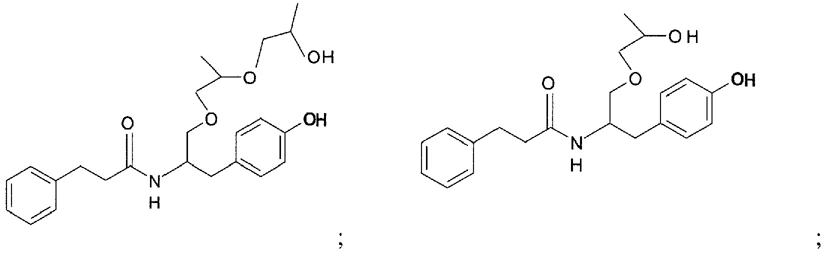 and 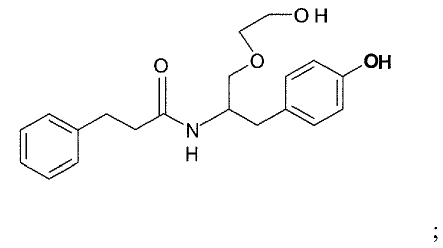 or a chiral, diastereomeric, racemic or geometric isomeric form thereof.

2. A compound for use as claimed in claim 1 for the treatment of acute or chronic pain.

3. A compound for use as claimed in claim 1 for the treatment of nociceptive pain or neuropathic pain.

4. A pharmaceutical composition for use in the treatment or prophylaxis of pain, said composition comprising a pharmaceutically effective amount of one or more of the compounds as claimed in claim 1, optionally together with one or more pharmaceutically acceptable excipients.

5. A pharmaceutical composition for use as claimed in claim 4, which composition comprises said one or more compounds in substantially pure form, said substantially pure form consisting of at least 95% wt. of said one or more compounds and up to 5% wt. of free polyalkylene glycol, with the total amount in said form of said one or more compounds and said free polyalkylene glycol being 100% wt.

6. A pharmaceutical composition for use as claimed in claim 4, which composition comprises said one or more compounds in partially pure form, said partially pure form consisting of about 5-60% wt. of the one or more compounds and about 95-40% wt. of free polyalkylene glycol, the total amount being 100% wt.

7. A pharmaceutical composition for use as claimed in any of claims 4-6, which is formulated for oral administration.

8. A pharmaceutical composition for use as claimed in any of claims 4-7, wherein said composition is formulated as a unit dosage form, preferably comprising from 0.1 to about 500 mg of the one or more compounds, more preferably comprising 1 to 300mg, more preferably 1 to 50mg, most preferably 25 to 50mg of one or more compounds.

9. A compound for use in the treatment or prophylaxis of inflammation, wherein the compound is selected from the group consisting of 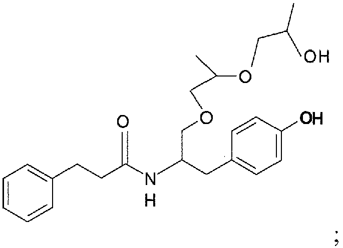 and 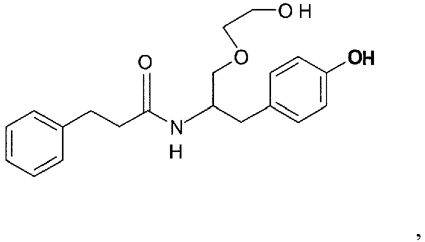 or a chiral, diastereomeric, racemic or geometric isomeric form thereof.

10. Use of a compound for the manufacture of a medicament for the treatment or prophylaxis of pain, wherein the compound is selected from the group consisting of 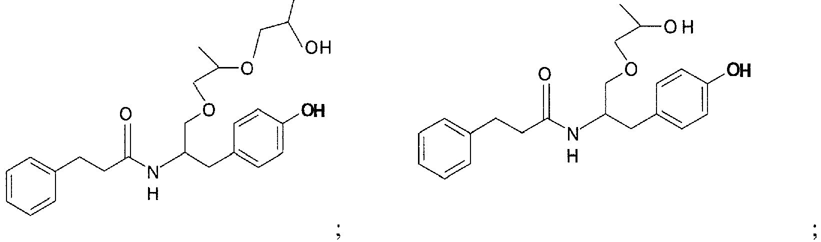 and 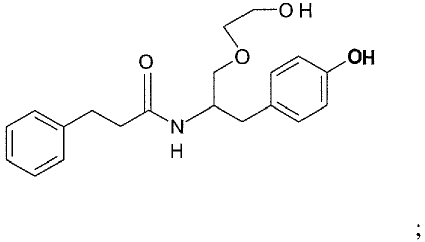 or a chiral, diastereomeric, racemic or geometric isomeric form thereof.

## Patentansprüche

1. Verbindung zur Verwendung bei der Behandlung oder Prophylaxe von Schmerzen, wobei die Verbindung ausgewählt ist aus der Gruppe bestehend aus 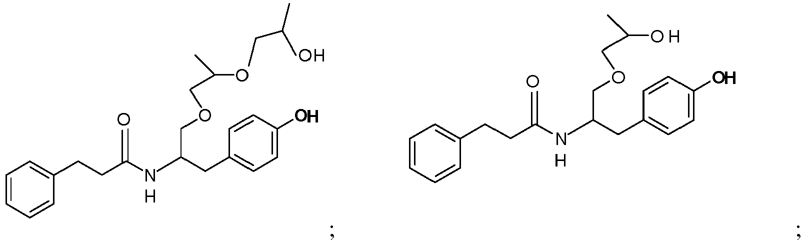 und 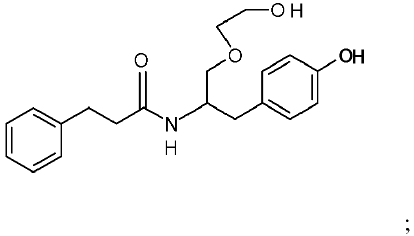 oder eine chirale, diastereomere, racemische oder geometrisch isomere Form davon.

2. Verbindung zur Verwendung nach Anspruch 1 zur Behandlung von akuten oder chronischen Schmerzen.

3. Verbindung zur Verwendung nach Anspruch 1 zur Behandlung von nozizeptiven Schmerzen oder neuropathischen Schmerzen.

4. Pharmazeutische Zusammensetzung zur Verwendung bei der Behandlung oder Prophylaxe von Schmerzen, wobei die genannte Zusammensetzung eine pharmazeutisch wirksame Menge von einer oder mehreren der Verbindungen nach Anspruch 1 umfasst, optional zusammen mit einem oder mehreren pharmazeutisch akzeptablen Hilfsstoffen.

5. Pharmazeutische Zusammensetzung zur Verwendung nach Anspruch 4, wobei die Zusammensetzung die eine oder mehreren genannten Verbindungen in im Wesentlichen reiner Form umfasst, wobei die genannte im Wesentlichen reine Form aus wenigstens 95 Gew.-% der einen oder mehreren genannten Verbindungen und bis zu 5 Gew.-% freiem Polyalkylenglykol besteht, wobei die Gesamtmenge der einen oder mehreren genannten Verbindungen und des genannten freien Polyalkylenglykols in der genannten Form 100 Gew.-% beträgt.

6. Pharmazeutische Zusammensetzung zur Verwendung nach Anspruch 4, wobei die Zusammensetzung die eine oder mehreren genannten Verbindungen in teilweise reiner Form umfasst, wobei die genannte teilweise reine Form aus etwa 5-60 Gew.-% der einen oder mehreren Verbindungen und etwa 95-40 Gew.-% freiem Polyalkylenglykol besteht, wobei die Gesamtmenge 100 Gew.-% beträgt.

7. Pharmazeutische Zusammensetzung zur Verwendung nach einem der Ansprüche 4-6, die zur oralen Verabreichung formuliert ist.

8. Pharmazeutische Zusammensetzung zur Verwendung nach einem der Ansprüche 4-7, wobei die genannte Zusammensetzung als Einheitsdosierungsform formuliert ist, die vorzugsweise 0,1 bis etwa 500 mg der ein oder mehreren Verbindungen umfasst, bevorzugter 1 bis 300 mg, bevorzugter 1 bis 50 mg, am bevorzugtesten 25 bis 50 mg der ein oder mehreren Verbindungen umfasst.

9. Verbindung zur Verwendung bei der Behandlung oder Prophylaxe einer Entzündung, wobei die Verbindung ausgewählt ist aus der Gruppe bestehend aus 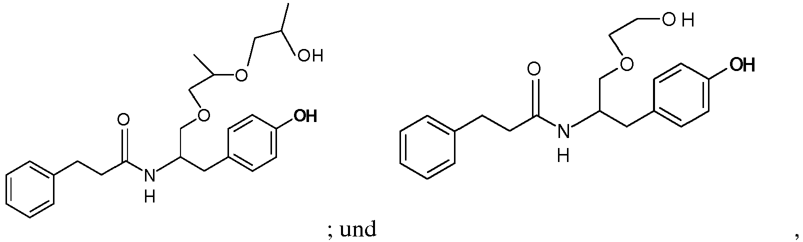 oder eine chirale, diastereomere, racemische oder geometrisch isomere Form davon.

10. Verwendung einer Verbindung zur Herstellung eines Medikaments zur Behandlung oder Prophylaxe von Schmerzen, wobei die Verbindung ausgewählt ist aus der Gruppe bestehend aus 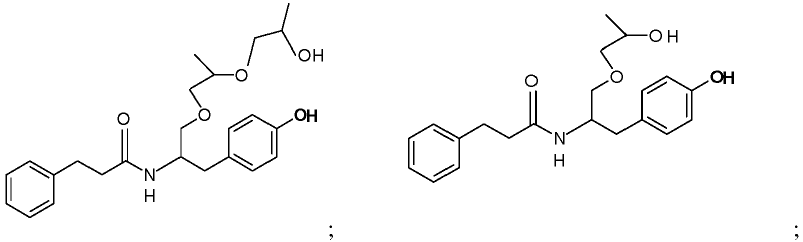 und 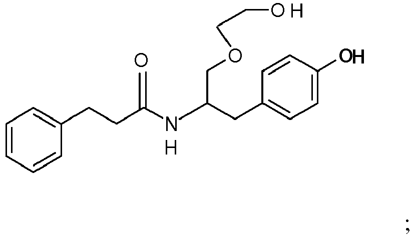 oder eine chirale, diastereomere, racemische oder geometrisch isomere Form davon.

## Revendications

1. Composé destiné à une utilisation dans le traitement ou la prophylaxie de la douleur, le composé étant sélectionné dans le groupe constitué de 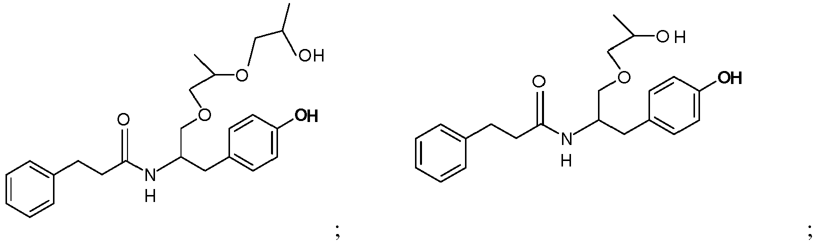 et 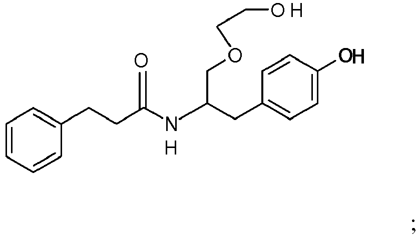 ou une forme isomère chirale, diastéréomère, racémique ou géométrique de celui-ci.

2. Composé destiné à une utilisation selon la revendication 1 pour le traitement d'une douleur aiguë ou chronique.

3. Composé destiné à une utilisation selon la revendication 1 pour le traitement d'une douleur nociceptive ou d'une douleur neuropathique.

4. Composition pharmaceutique destinée à une utilisation dans le traitement ou la prophylaxie de la douleur, ladite composition comprenant une quantité pharmaceutiquement efficace d'un ou plusieurs des composés selon la revendication 1, optionnellement conjointement avec un ou plusieurs excipients pharmaceutiquement acceptables.

5. Composition pharmaceutique destinée à une utilisation selon la revendication 4, ladite composition comprenant lesdits un ou plusieurs composés sous une forme quasiment pure, ladite forme quasiment pure consistant en au moins 95 % en poids desdits un ou plusieurs composés et jusqu'à 5 % en poids de polyalkylène glycol libre, la quantité totale sous ladite forme desdits un ou plusieurs composés et dudit polyalkylène glycol libre étant de 100 % en poids.

6. Composition pharmaceutique destinée à une utilisation selon la revendication 4, ladite composition comprenant lesdits un ou plusieurs composés sous une forme partiellement pure, ladite forme partiellement pure consistant en environ 5 à 60 % en poids desdits un ou plusieurs composés et environ 95 à 40 % en poids de polyalkylène glycol libre, la quantité totale étant de 100 % en poids.

7. Composition pharmaceutique destinée à une utilisation selon l'une quelconque des revendications 4 à 6, laquelle est formulée pour une administration orale.

8. Composition pharmaceutique destinée à une utilisation selon l'une quelconque des revendications 4 à 7, ladite composition étant formulée comme une forme galénique unitaire, comprenant préférablement de 0,1 à environ 500 mg desdits un ou plusieurs composés, comprenant plus préférablement 1 à 300 mg, plus préférablement 1 à 50 mg, plus préférablement 25 à 50 mg desdits un ou plusieurs composés.

9. Composé destiné à une utilisation dans le traitement ou la prophylaxie d'une inflammation, le composé étant sélectionné dans le groupe constitué de 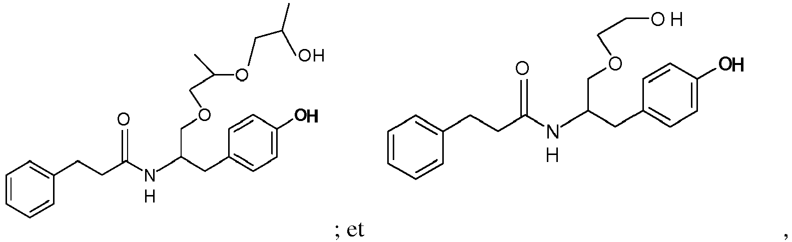 ou une forme isomère chirale, diastéréomère, racémique ou géométrique de celui-ci.

10. Utilisation d'un composé pour la fabrication d'un médicament pour le traitement ou la prophylaxie de la douleur, le composé étant sélectionné dans le groupe constitué de 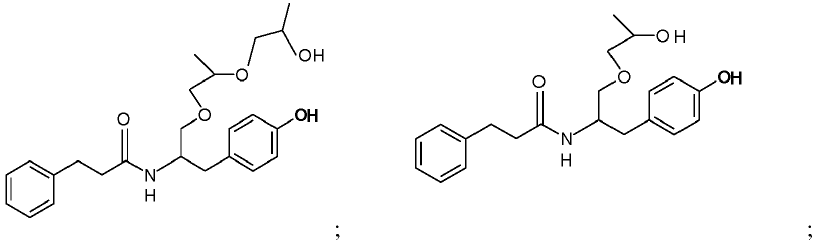 et 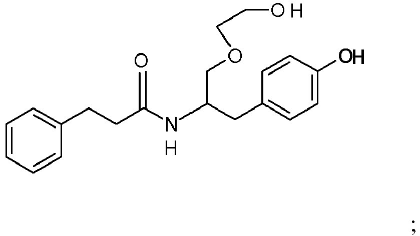 ou une forme isomère chirale, diastéréomère, racémique ou géométrique de celui-ci.
